# EUROPEAN PATENT SPECIFICATION

(11) **EP 4 186 551 B1**
(45) Date of publication and mention of the grant of the patent: **06.08.2025**
(21) Application number: 21845472.6
(22) Date of filing: 07.06.2021
(51) Int. Cl.: A61M 16/16, A61M 16/10

(54) **HUMIDIFIER AND VENTILATION TREATMENT DEVICE**
BEFEUCHTER UND BEATMUNGSBEHANDLUNGSVORRICHTUNG
HUMIDIFICATEUR ET DISPOSITIF DE TRAITEMENT PAR VENTILATION

(30) Priority: 24.07.2020 CN 202010724554
(43) Date of publication of application: 31.05.2023
(73) Proprietor: BMC (Tianjin) Medical Co., Ltd., Tianjin 301700 (CN)
(72) Inventor: LIU, Lijun, Tianjin 301700 (CN); ZHI, Jianxin, Tianjin 301700 (CN); ZHUANG, Zhi, Tianjin 301700 (CN)
(74) Representative: Grünecker Patent- und Rechtsanwälte PartG mbB
(86) International application number: PCT/CN2021/098705
(87) International publication number: WO 2022/017019

(56) References cited:
- WO-A1-2020/032808
- CN-A- 108 283 753
- CN-A- 110 594 924
- CN-A- 111 939 421
- CN-U- 203 907 823
- CN-U- 205 980 133
- CN-U- 205 980 133
- US-A1- 2011 288 474
- US-A1- 2019 134 342

## Description

### CROSS REFERENCE TO RELEVANT APPLICATIONS

The present disclosure claims the priority of the Chinese patent application filed on July 24th, 2020 before the Chinese Patent Office with the application number of 202010724554.1 and the title of "VENTILATION TREATMENT DEVICE".

### TECHNICAL FIELD

The present disclosure relates to the technical field of medical devices, and particularly relates to a humidifier and a ventilation-treatment device.

### BACKGROUND

Ventilation-treatment devices, for example, breathing machines, are commonly used medical devices, and may facilitate patients to increase the pulmonary ventilation capacity, whereby improving the respiratory function of the patients, and saving the storage capacity of the heart and the lung of the patients. They are generally used in apnea syndrome, dyspnea, serious ventilation disorder, neuromuscular paralysis, asphyxia, cardio-pulmonary resuscitation and so on. In the prior art, the operation mode of all of the ventilation-treatment devices, for example, breathing machines, is to control a fan to operate to output gas of a certain pressure, and after being humidified through the humidifier, the gas reaches the patient side for the patient to use.

The humidifier is an important component of ventilation-treatment devices, for example, breathing machines. Its function is to heat and humidify the sucked gas. An excellent effect of heating and humidification may prevent and reduce secondary infection of the respiratory tract of the patient and irritation to the cardiopulmonary system caused by the mechanical ventilation, to maintain the alveoli wet. Moreover, that may reduce the consumption of the heat and the water in the respiratory tract, to prevent the generation of sputum scab in the air passage, and may reduce the viscosity of the secreta, to facilitate sputum excretion. Examples of such humidifiers are disclosed in US 2019/134342, CN 205 980 133, WO 2020/032808.

However, the humidifiers of many of the conventional ventilation-treatment devices have the problem of dry heating. For example, when the patient requires continuously wearing the breathing machine at night for a long time, because of the selection of the humidification level, the water storage capacity of the water tank and the difference in the usage durations, the water tank might lack water in the second half period of the usage, which results in dry heating. Moreover, dry heating results in the following problems and risks. Dry heating causes that the heating region of the humidifier is in a high-temperature state for a long time, and the heating region and the sealing components adjacent to it are aged quickly, which might result in water leakage, to cause damaging of the electric elements by short circuiting, to affect the life of the product. In dry heating, the breathing machine is still in the operating state, at which point there is no water in the water tank, and the breathing machine delivers the dry high-temperature gas to the patient side, which might result in risks such as high-temperature ambustion of the respiratory tract of the patient.

### SUMMARY

The invention is defined by the appended claims. Subject-matter, referred as embodiments, disclosures and/or aspects, which does not fall under the scope of the claims is not part of the invention.

The present disclosure provides a humidifier and a ventilation-treatment device, to solve the problem of the humidifier of the ventilation-treatment devices in the prior art that the risk of dry heating exists, which affects the life of the product, and results in ambustion of the patient.

In a first aspect of the embodiments of the present invention, there is provided a humidifier as defined in claim 1.

Optionally, at least part of the water adsorbing filter elements incline relatively to a transverse direction of the humidifying chamber, and extend in an inclined direction from a top to a bottom of the humidifying chamber to form the first cross section.

Optionally, the water adsorbing filter elements inclining relatively to the transverse direction of the humidifying chamber include a plurality of first water adsorbing filter elements and a plurality of second water adsorbing filter elements that are arranged alternately inside the humidifying chamber in the gas-flow direction, and directions of inclining relative to the transverse direction of the humidifying chamber of the first water adsorbing filter elements and the second water adsorbing filter elements are opposite.

Optionally, neighboring instances of the first water adsorbing filter elements and the second water adsorbing filter elements form a V shape therebetween.

Optionally, a first water adsorbing filter element disposed at a first position in the gas-flow direction and a second water adsorbing filter element disposed at a last position in the gas-flow direction individually extend in the direction from the top to the bottom of the humidifying chamber to form the first cross section; and
the first water adsorbing filter elements and the second water adsorbing filter elements are arranged alternately between the first water adsorbing filter element disposed at the first position and the second water adsorbing filter element disposed at the last position.

Optionally, lengths of extension in the direction from the top to the bottom of the humidifying chamber of part or all of the water adsorbing filter elements are unequal.

Optionally, the lengths of the extension in the direction from the top to the bottom of the humidifying chamber of the part or all of the water adsorbing filter elements decrease or increase sequentially in an arrangement direction.

Optionally, each two neighboring instances of the first water adsorbing filter elements and the second water adsorbing filter elements are grouped as one group, and each of the remaining individual water adsorbing filter elements is grouped as one group; and
lengths of extension in the direction from the top to the bottom of the humidifying chamber of different groups of the water adsorbing filter elements decrease or increase sequentially in the arrangement direction.

Optionally, the bottom of the humidifying chamber inclines, and each of the water adsorbing filter elements extends in an extension direction from the top to the bottom of the humidifying chamber.

Optionally, each of the water adsorbing filter elements includes a water-absorbing-fiber matrix and a border frame for fixing the water-absorbing-fiber matrix; and
an installation region for installing the water adsorbing filter elements is provided inside the humidifying chamber.

Optionally, the humidifier further includes:
a water tank, wherein the water tank is provided at a bottom of the housing, a first communicating component is provided at a top of the water tank, a second communicating component in cooperation with the first communicating component is provided at the bottom of the housing, and the housing and the water tank are communicated by the cooperation between the first communicating component and second communicating component.

Optionally, the humidifier further includes:
a first controller, wherein the first controller is electrically connected to the monitoring device, and is for, based on the gas property monitored by the monitoring device, controlling an operation state of the humidifier, and/or, an operation state of a ventilation-treatment-device mainframe in communication with the humidifier.

In the second aspect of the embodiments of the present disclosure, there is further provided a ventilation-treatment device including a mainframe for generating a gas of a preset pressure, the mainframe includes a gas outlet channel, and the ventilation-treatment device further includes: the humidifier stated above, and the gas outlet channel of the mainframe is in communication with the gas inlet of the humidifier.

Optionally, the ventilation-treatment device further includes:
a second controller, wherein the second controller is electrically connected to the monitoring device, is electrically connected to the mainframe and/or the humidifier, and is for, based on the gas property monitored by the monitoring device, controlling an operation state of the mainframe and/or the humidifier.

Optionally, the ventilation-treatment device further includes:
a first heating body, wherein the first heating body is located upstream of the humidifier.

As compared with the prior art, the present disclosure has the following advantages:

In the embodiments of the present disclosure, the humidifier includes a housing, the housing is provided with a gas inlet, a gas outlet and a humidifying chamber for containing a humidifying liquid, and an external gas enters the housing via the gas inlet, flows through the humidifying chamber, and is discharged via the gas outlet. The humidifier further includes a water-absorbing-fiber device and a monitoring device, the water-absorbing-fiber device is provided inside the humidifying chamber, and extends in a longitudinal direction of the humidifying chamber, the water-absorbing-fiber device includes a first cross section, and the first cross section intersects with a gas-flow direction of the external gas after the external gas enters the humidifying chamber. In usage, the humidifying liquid may be placed inside the humidifying chamber. After the humidifying liquid contacts the water-absorbing-fiber device, because of the capillary effect, the humidifying liquid climbs along the water-absorbing-fiber device, whereby the water-absorbing-fiber device fully contains the humidifying liquid. The first cross section of the water-absorbing-fiber device intersects with the gas-flow direction. An external gas, after entering the humidifying chamber, may contact the water-absorbing-fiber device, and bring away the humidifying liquid in the water-absorbing-fiber device, to reach the effect of humidification. The monitoring device is provided downstream of the water-absorbing-fiber device in the gas-flow direction, and is for monitoring a gas property downstream of the water-absorbing-fiber device. When the liquid inside the humidifying chamber is insufficient, the water-absorbing-fiber device lacks the humidifying liquid, and the gas downstream of the water-absorbing-fiber device has an increased temperature and a reduced humidity. Therefore, by monitoring the gas property downstream of the water-absorbing-fiber device by using the monitoring device, a state of liquid insufficiency may be detected in time, to achieve the purpose of preventing dry heating. Accordingly, by providing the water-absorbing-fiber device and the monitoring device, dry heating of the humidifier may be effectively prevented without the patient or the user always paying attention to the residual amount of the humidifying liquid, which prevents component damage and patient ambustion caused by dry heating of the humidifier, thereby prolonging the service life of the product, and improving the safety and the reliability.

The above description is merely a summary of the technical solutions of the present disclosure. In order to more clearly know the elements of the present disclosure to enable the implementation according to the contents of the description, and in order to make the above and other purposes, features and advantages of the present disclosure more apparent and understandable, the particular embodiments of the present disclosure are provided below.

### BRIEF DESCRIPTION OF THE DRAWINGS

In order to more clearly illustrate the technical solutions of the embodiments of the present disclosure or the prior art, the figures that are required to describe the embodiments will be briefly described below.
FIG. 1 is a schematic diagram of the overall appearance of an embodiment of the humidifier according to the embodiments of the present disclosure;
FIG. 2 is a schematic exploded sectional structural diagram of an embodiment of the humidifier according to the embodiments of the present disclosure;
FIG. 3 is a schematic sectional structural diagram of the housing of the humidifier according to the present disclosure in FIG. 2;
FIG. 4 is a schematic structural diagram of an embodiment of a water adsorbing filter element of the humidifier according to the embodiments of the present disclosure;
FIG. 5 is a schematic diagram of the climbing of the humidifying liquid on the water adsorbing filter elements of the humidifier according to the present disclosure in FIG. 2.
FIG. 6 is a schematic diagram of the gas-flow direction and the climbing of the humidifying liquid on the water adsorbing filter elements in the normal state of the humidifier according to the present disclosure in FIG. 2;
FIG. 7 is a schematic diagram of the gas-flow direction and the climbing of the humidifying liquid on the water adsorbing filter elements in the water-lacking state of the humidifier according to the present disclosure in FIG. 2;
FIG. 8 is a schematic sectional structural diagram of a housing of another embodiment of the humidifier according to the embodiments of the present disclosure;
FIG. 9 is a schematic sectional structural diagram of a housing of yet another embodiment of the humidifier according to the embodiments of the present disclosure;
FIG. 10 is a schematic diagram of the gas-flow direction and the climbing of the humidifying liquid on the water adsorbing filter elements in the normal state of the humidifier according to the present disclosure in FIG. 9;
FIG. 11 is a schematic diagram of the gas-flow direction and the climbing of the humidifying liquid on the water adsorbing filter elements in the water-lacking state of the humidifier according to the present disclosure in FIG. 9;
FIG. 12 is another schematic diagram of the gas-flow direction and the climbing of the humidifying liquid on the water adsorbing filter elements in the water-lacking state of the humidifier according to the present disclosure in FIG. 9;
FIG. 13 is a schematic sectional structural diagram of a housing and a standby water tank of another embodiment of the humidifier according to the embodiments of the present disclosure;
FIG. 14 is a schematic diagram of the water level of the standby water tank in the normal state of the humidifier according to the present disclosure in FIG. 13;
FIG. 15 is a schematic diagram of the water level of the standby water tank in the water-lacking state of the humidifier according to the present disclosure in FIG. 13;
FIG. 16 is a schematic sectional structural diagram of a housing of still another embodiment of the humidifier according to the embodiments of the present disclosure;
FIG. 17 is a schematic structural diagram of another embodiment of water adsorbing filter elements according to the embodiments of the present disclosure;
FIG. 18 is a schematic diagram of the gas-flow direction and the climbing of the humidifying liquid on the water adsorbing filter elements in the normal state of the humidifier in FIG. 16;
FIG. 19 is a schematic diagram of the gas-flow direction and the climbing of the humidifying liquid on the water adsorbing filter elements in the water-lacking state of the humidifier in FIG. 16;
FIG. 20 is a schematic sectional structural diagram of a housing of yet another embodiment of the humidifier according to the embodiments of the present disclosure;
FIG. 21 is a schematic diagram of the gas-flow direction and the climbing of the humidifying liquid on the water adsorbing filter elements in the normal state of the humidifier in FIG. 20;
FIG. 22 is a schematic diagram of the gas-flow direction and the climbing of the humidifying liquid on the water adsorbing filter elements in the water-lacking state of the humidifier in FIG. 20;
FIG. 23 is a schematic sectional structural diagram of a housing and a standby water tank of yet another embodiment of the humidifier according to the embodiments of the present disclosure;
FIG. 24 is a schematic diagram of the water level of the standby water tank in the normal state of the humidifier in FIG. 23;
FIG. 25 is a schematic diagram of the water level of the standby water tank in the water-lacking state of the humidifier in FIG. 23;
FIG. 26 is a schematic structural diagram of another embodiment of the humidifier according to the embodiments of the present disclosure;
FIG. 27 is a cross-sectional view taken in the radial direction of the humidifier in FIG. 26;
FIG. 28 is a schematic structural diagram of the interior of the housing of the humidifier in FIG. 26;
FIG. 29 is a schematic structural diagram of the interior of the humidifier in FIG. 26, wherein part of the top cover of the humidifier is omitted, and the arrows in the figure show the flowing direction in which the gas flow flows through the humidifier;
FIG. 30 is a schematic structural diagram of an embodiment of the ventilation-treatment device according to the embodiments of the present disclosure;
FIG. 31 is a schematic structural diagram of another embodiment of the ventilation-treatment device according to the embodiments of the present disclosure;
FIG. 32 is a schematic exploded structural diagram of the ventilation-treatment device in FIG. 31;
FIG. 33 is a schematic diagram showing the cooperation relation between the mainframe and the first heating body of the ventilation-treatment device in FIG. 32;
FIG. 34 is a schematic diagram from another visual angle showing the cooperation relation between the mainframe and the first heating body of the ventilation-treatment device in FIG. 32;
FIG. 35 is a partially sectional view of the ventilation-treatment device in FIG. 31, which shows the mainframe, and the first heating body and a switching unit that are installed on the mainframe;
FIG. 36 is a partially top sectional view of the ventilation-treatment device in FIG. 31, which shows the gas-flow channel of the device, wherein the arrows show the flowing direction of the gas flow;
FIG. 37 is a schematic structural diagram of a ventilation-treatment device according to another embodiment of the present disclosure;
FIG. 38 is a schematic exploded structural diagram of the ventilation-treatment device in FIG. 37;
FIG. 39 is a partially top sectional view of the ventilation-treatment device in FIG. 37, wherein the arrows show the flowing direction of the gas;
FIG. 40 is a schematic diagram of the installation of the second heating body of the ventilation-treatment device in FIG. 39 in an outlet channel;
FIG. 41 is a schematic structural diagram of the mainframe of the ventilation-treatment device in FIG. 37;
FIG. 42 is a schematic structural diagram of a second heating body that can be correspondingly installed into the mainframe shown in FIG. 41; and
FIG. 43 is a schematic flow chart of a controlling method of the humidifier according to the embodiments of the present disclosure.

Description of the reference numbers:
1 mainframe; 11 first contact-point connecting end; 12 second contact-point connecting end; 13 gas returning chamber; 14 clipping rib; 2 first heating body; 21 first-heating-body contact point; 3 switching unit; 31 hard frame; 32 soft body; 321 first convex rib; 322 second convex rib; 4 humidifier; 41 housing; 411 second communicating holes; 42 water adsorbing filter elements; 421 first cross section; 422 first water adsorbing filter elements; 423 second water adsorbing filter elements; 424 water adsorbing filter element disposed at the first position; 425 water adsorbing filter element disposed at the last position; 426 water-absorbing-fiber matrix; 427 border frame; 43 gas inlet; 44 gas outlet; 45 humidifying chamber; 451 water-adsorbing-filter-element installing units; 452 blocking plates; 453 installation region; 5 connecting pipeline; 6 patient side; 7 second heating body; 71 slot; 72 second-heating-body contact point; 73 sealing rib; 8 second monitor; 9 monitoring device; 91 third monitors; 10 standby water tank; 61 first communicating holes; and 100 ventilation-treatment device

### DETAILED DESCRIPTION

The technical solutions of the embodiments of the present disclosure will be clearly and completely described below with reference to the drawings of the embodiments of the present disclosure. Apparently, the described embodiments are merely certain embodiments of the present disclosure, rather than all of the embodiments. All of the other embodiments that a person skilled in the art obtains on the basis of the embodiments of the present disclosure without paying creative work fall within the protection scope of the present disclosure.

The humidifier and the ventilation-treatment device according to the present disclosure will be described in detail below with reference to particular embodiments.

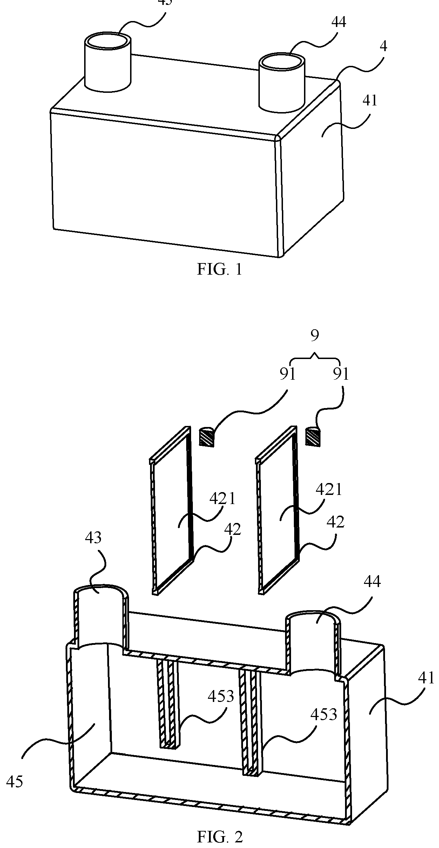

Referring to FIGs. 1-25, an embodiment of the present disclosure provides a humidifier 4. The humidifier 4 includes a housing 41, the housing 41 is provided with a gas inlet 43, a gas outlet 44 and a humidifying chamber 45 for containing a humidifying liquid, and an external gas enters the housing 41 via the gas inlet 43, flows through the humidifying chamber 45, and is discharged via the gas outlet 44. The humidifier 4 further includes:
a water-absorbing-fiber device, wherein the water-absorbing-fiber device is provided inside the humidifying chamber 45, and extends in the longitudinal direction of the humidifying chamber 45, the water-absorbing-fiber device includes a first cross section 421, and the first cross section 421 intersects with the gas-flow direction of the external gas after the external gas enters the humidifying chamber 45; and
a monitoring device 9, wherein the monitoring device 9 is provided downstream of the water-absorbing-fiber device in the gas-flow direction, and is for monitoring a gas property downstream of the water-absorbing-fiber device.

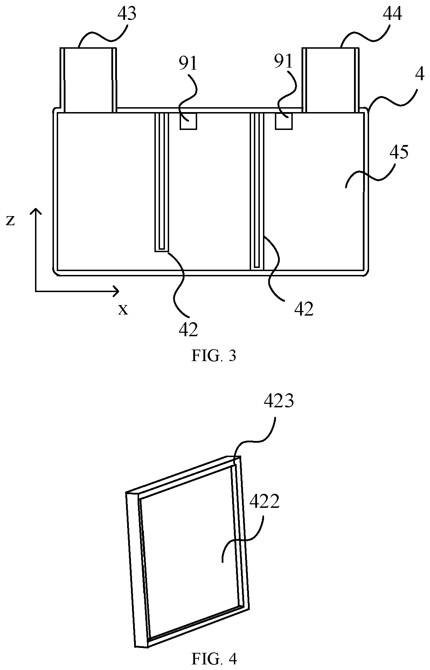

The longitudinal direction of the humidifying chamber 45 refers to the direction of the chamber depth of the humidifying chamber 45, for example, the direction of the Z-axis in FIG. 3. The transverse direction of the humidifying chamber 45 refers to the direction transverse to the chamber of the humidifying chamber 45, for example, the direction of the X-axis in FIG. 3.

When the humidifier 4 according to the embodiments of the present disclosure is being used, the gas outlet channel of the ventilation-treatment device may be communicated with the gas inlet 43 of the humidifier 4. After the humidifying liquid (for example, water) is placed inside the humidifying chamber 45 and the humidifying liquid contacts the water-absorbing-fiber device, because of the capillary effect, the humidifying liquid climbs along the water-absorbing-fiber device, whereby the water-absorbing-fiber device fully contains the humidifying liquid. The first cross section 421 of the water-absorbing-fiber device intersects with the gas-flow direction. When the ventilation-treatment device is in the normal ventilation-treatment state, the gas enters the humidifying chamber 45 of the housing 41 via the gas inlet 43, contacts the water-absorbing-fiber device, and brings away the humidifying liquid thereon, to reach the effect of humidification. When the liquid inside the humidifying chamber 45 is insufficient, the water-absorbing-fiber device lacks the humidifying liquid, the gas cannot reach the effect of humidification when passing through the water-absorbing-fiber device, and the gas downstream of the water-absorbing-fiber device, as compared with the state not lacking the humidifying liquid, has an increased temperature and a reduced humidity. Therefore, by monitoring the gas property downstream of the water-absorbing-fiber device by using the monitoring device 9, a state of liquid insufficiency may be detected in time, to achieve the purpose of preventing dry heating.

Accordingly, by providing the water-absorbing-fiber device and the monitoring device 9, dry heating of the humidifier 4 may be effectively prevented without the patient or the user always paying attention to the residual amount of the humidifying liquid, which prevents component damage and patient ambustion caused by dry heating of the humidifier 4, thereby prolonging the service life of the product, and improving the safety and the reliability.

The position of the heating component of the humidifier 4 is not limited in the embodiments of the present disclosure. For example, the heating component may be provided at the bottom of the humidifier 4.

The housing 41 may include an upper cover and a lower housing, and the upper cover is hinged to the lower housing, to facilitate the replacement when it is required to replace the water-absorbing-fiber device.

As an alternative embodiment, as shown in FIGs. 2 and 16-17, the water-absorbing-fiber device includes:
at least two water adsorbing filter elements 42, wherein the at least two water adsorbing filter elements 42 are arranged inside the humidifying chamber 45 sequentially in the gas-flow direction, and each of the water adsorbing filter elements 42 has the first cross section 421.

Here, two or more water adsorbing filter elements 42 may be provided inside the humidifying chamber 45, and sequentially arranged in the direction of the flowing of the external gas inside the humidifying chamber 45, and each of the water adsorbing filter elements 42 has the first cross section 421 intersecting with the gas-flow direction. Accordingly, after the external gas enters the humidifying chamber 45, it sequentially passes through the plurality of sequentially arranged water adsorbing filter elements 42, contacts the water adsorbing filter elements 42, and brings away the humidifying liquid thereon.

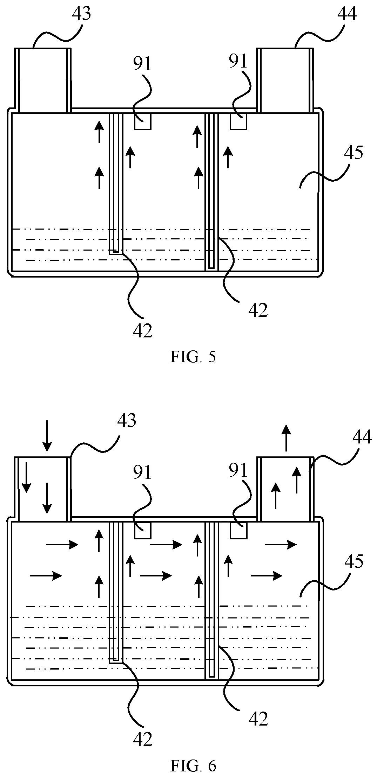

For example, as shown in FIG. 3, two water adsorbing filter elements 42 may be provided inside the humidifying chamber 45, and the two water adsorbing filter elements 42 are sequentially arranged in the gas-flow direction. As shown in FIG. 5, after the humidifying liquid is placed inside the humidifying chamber 45, because of the capillary effect, the humidifying liquid climbs along the water adsorbing filter elements 42, whereby the water adsorbing filter elements 42 fully contain the humidifying liquid. As shown in FIG. 6, when the ventilation-treatment device, for example, a breathing machine, is in the normal ventilation-treatment state, after the gas enters the humidifying chamber 45 via the gas inlet 43, it sequentially passes through the two water adsorbing filter elements 42, and brings the warm and wet gas to the patient side via the gas outlet 44 for the patient to use.

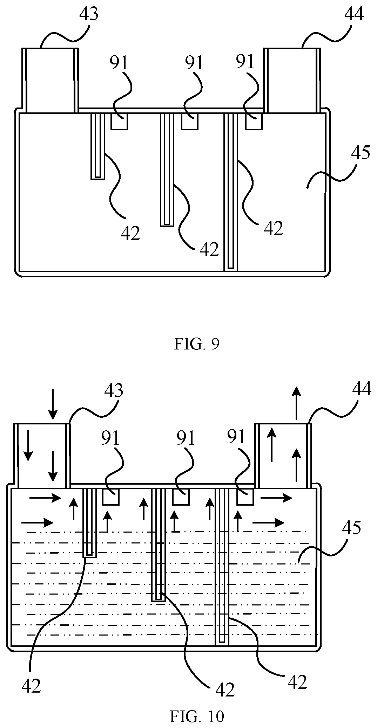

As another example, as shown in FIG. 9, three water adsorbing filter elements 42 may also be provided inside the humidifying chamber 45, and the three water adsorbing filter elements 42 are sequentially arranged in the gas-flow direction. As shown in FIG. 10, after the humidifying liquid is placed inside the humidifying chamber 45, because of the capillary effect, the humidifying liquid climbs along the water adsorbing filter elements 42, whereby the water adsorbing filter elements 42 fully contain the humidifying liquid. When the ventilation-treatment device, for example, a breathing machine, is in the normal ventilation-treatment state, after the gas enters the humidifying chamber 45 via the gas inlet 43, it sequentially passes through the three water adsorbing filter elements 42, and brings the warm and wet gas to the patient side via the gas outlet 44 for the patient to use.

In this case, if the humidifying chamber 45 does not lack the humidifying liquid, at least two of the water adsorbing filter elements 42 fully contain the humidifying liquid, and the gas flow sequentially passes through the at least two water adsorbing filter elements 42 to bring away the humidifying liquid, which may reach the effect of humidification, and, by the multilayer humidification, may effectively increase the humidification rate.

Optionally, in the embodiments shown in FIGs. 2-15, each of the water adsorbing filter elements 42 extends in the direction from the top to the bottom of the humidifying chamber, and the first cross section 421 intersects with the gas-flow direction perpendicularly.

In this case, the first cross section 421 of the water adsorbing filter elements 42 completely penetrates a second cross section inside the humidifying chamber 45 between the gas inlet 43 and the gas outlet 44 in the orthogonal direction. The gas, when passing through the water adsorbing filter elements 42, may contact the entire surface of the water adsorbing filter elements 42, and the installation is more convenient.

The water adsorbing filter elements 42 may also be configured as a folded shape, to increase the water adsorbing area of the water adsorbing filter elements 42, and further increase the contact area between the gas and the water adsorbing filter elements 42, thereby increasing the humidification efficiency.

As an alternative embodiment, as shown in FIG. 4, each of the water adsorbing filter elements 42 may include a water-absorbing-fiber matrix 426 and a border frame 427 for fixing the water-absorbing-fiber matrix 426.

The water-absorbing-fiber matrix 426 is a soft material, and has excellent water absorbency and gas permeability. After the water-absorbing-fiber matrix 426 contacts the humidifying liquid, it may quickly absorb the humidifying liquid.

The fixing border frame 427 is provided at the periphery of the water-absorbing-fiber matrix 426, and may encapsulate and fix the water-absorbing-fiber matrix 426, to enable it to be stable, to facilitate the installation and the usage.

As shown in FIG. 2, an installation region 453 for installing the water adsorbing filter elements 42 is provided inside the humidifying chamber 45.

In this case, the water adsorbing filter elements 42 are a replaceable consumable. By the cooperation between the installation region 453 and the border frame 423 of the water adsorbing filter element 42, the water adsorbing filter element 42 may be conveniently installed or detached.

As an alternative embodiment, the monitoring device 9 includes:
at least two third monitors 91, wherein the third monitors 91 are provided downstream of different water adsorbing filter elements 42 in the gas-flow direction.

Here, each of the water adsorbing filter elements 42 may be provided with one third monitor 91 downstream, to monitor the downstream gas property. When the gas property monitored by any one of the third monitors 91 is abnormal, a state of liquid insufficiency may be detected in time, to achieve the purpose of preventing dry heating. In this case, the multilayer monitoring improves the safety.

The gas property monitored by the third monitors 91 may be the temperature and/or the humidity of the gas. When the gas entering the humidifier 4 is a heated dry air, that has a better effect for the humidification.

As an alternative embodiment, the lengths of the extension in the direction from the top to the bottom of the humidifying chamber 45 of part or all of the water adsorbing filter elements 42 are unequal.

Here, at least two water adsorbing filter elements 42 of unequal lengths may be provided inside the humidifying chamber 45. The water adsorbing filter element 42 of the longer length may extend to the bottom of the humidifying chamber 45 or a position adjacent to the bottom, and merely when the humidifying liquid inside the humidifying chamber 45 has a very low residual amount and there is a serious liquid lacking, the situation emerges in which the water adsorbing filter element 42 cannot absorb the humidifying liquid, the temperature of the downstream gas increases and the humidity decreases. Moreover, the water adsorbing filter element 42 of the shorter length extends to the midstream, downstream or a higher position in the humidifying chamber 45, and when the humidifying liquid inside the humidifying chamber 45 has a low residual amount, the situation emerges in which the water adsorbing filter element 42 cannot absorb the humidifying liquid, the temperature of the downstream gas increases and the humidity decreases. Therefore, by providing at least two water adsorbing filter elements 42 of unequal lengths inside the humidifying chamber 45, and providing one third monitor 91 downstream for each of the water adsorbing filter elements 42, the residual amounts of the humidifying liquid at different time periods may be monitored in detail, to adjust in time the humidification level based on the monitoring result, to reasonably plan the heating temperature and duration for the patient for long-time and continuous usage of the ventilation-treatment device.

It may be configured that the lengths of the extension in the direction from the top to the bottom of the humidifying chamber 45 of the part or all of the water adsorbing filter elements 42 decrease or increase sequentially in the arrangement direction.

In this case, by providing inside the humidifying chamber 45 at least two water adsorbing filter elements 42 whose lengths sequentially decrease or increase, and providing one third monitor 91 downstream for each of the water adsorbing filter elements 42, liquid lacking of different degrees inside the humidifying chamber 45 may be monitored, to control the humidifier 4 according thereto, to prevent dry heating.

For example, in the embodiments shown in FIGs. 2-7, two water adsorbing filter elements 42 may be provided inside the humidifying chamber 45. The two water adsorbing filter elements 42 are sequentially arranged in the gas-flow direction, and are located between the gas inlet 43 and the gas outlet 44, and the lengths of the extension in the longitudinal direction of the humidifying chamber 45 of the two water adsorbing filter elements 42 sequentially increase in the arrangement direction. As shown in FIG. 5, after the two water adsorbing filter elements 42 are installed within the installation region of the water adsorbing filter elements, when the humidifying liquid in the humidifying chamber 45 is at the normal-operating-state water level, the depths of the soaking of the water adsorbing filter elements 42 into the humidifying liquid inside the humidifying chamber 45 are unequal, and, according to the principle of capillary effect, the humidifying liquid climbs along the water absorbing fiber till the water absorbing fiber fully contains water. The two third monitors 91 are located behind the two water adsorbing filter elements 42, and are used for monitoring the gas property (for example, temperature and/or humidity) after the gas passes through the water adsorbing filter elements 42. As shown in FIG. 6, when the ventilation-treatment device is in the normal treatment state, the gas, after entering the humidifying chamber 45 via the gas inlet 43 of the housing 41, sequentially passes through the two water adsorbing filter elements 42, and brings the warm and wet gas to the patient side via the gas outlet 44 for the patient to use, at which point both of the two water adsorbing filter elements 42 fully contain the humidifying liquid, and both of the two third monitors 91 collect the gas property of the normal operating state.

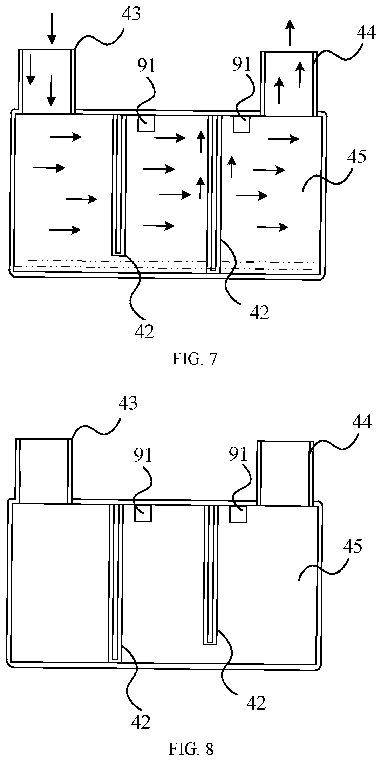

As shown in FIG. 7, when the humidifying liquid in the humidifying chamber 45 is insufficient or lacked, the second water adsorbing filter element 42 is closer to the liquid surface, and still fully contains the humidifying liquid, while the first water adsorbing filter element 42 has already disengaged from the liquid surface, in which the water absorbing fiber contains no or little humidifying liquid. At this point, the gas entering the gas inlet 43 may merely bring away the humidifying liquid from the second water adsorbing filter element 42, and cannot bring away the humidifying liquid or brings away a small amount of the humidifying liquid from the first water adsorbing filter element 42. Therefore, the gas property collected by the second third monitor 91 is substantially the same as that of the normal operating state, while, in the gas property collected by the first third monitor 91, as compared with the normal operating state, the temperature increases and the humidity decreases. The third monitors 91, after collecting the gas property, may emit an instruction to a controlling system, to reduce the heating power of the humidifier 4 or stop the heating of the humidifier 4, to prevent dry heating of the humidifier 4.

In the embodiment shown in FIG. 8, the positions of the two water adsorbing filter elements 42 of the unequal lengths in FIG. 5 may be exchanged; in other words, the water adsorbing filter element 42 further from the liquid surface is close to the gas outlet 44, and the water adsorbing filter element 42 closer to the liquid surface is close to the gas inlet 43. When the humidifying liquid in the humidifying chamber 45 is at the normal-operating-state water level, both of the two third monitors 91 behind the two water adsorbing filter elements 42 collect the gas property of the normal operating state.

When the humidifying liquid in the humidifying chamber 45 is insufficient or lacked, the second water adsorbing filter element 42 has already disengaged from the liquid surface, in which the water absorbing fiber contains no or little humidifying liquid, and the first water adsorbing filter element 42 is closer to the liquid surface, and still fully contains the humidifying liquid. Therefore, in the gas property collected by the second third monitor 91, as compared with the normal operating state, the temperature increases and the humidity decreases, while the gas property collected by the first third monitor 91 is substantially the same as that of the normal operating state. The third monitors 91, after collecting the gas property, may emit an instruction to a controlling system, to reduce the heating power of the humidifier 4 or stop the heating of the humidifier 4, to prevent dry heating of the humidifier 4.

In the embodiments shown in FIGs. 9-12, three water adsorbing filter elements 42 may be provided inside the humidifying chamber 45. The three water adsorbing filter elements 42 are sequentially arranged in the gas-flow direction, and the lengths of the extension in the longitudinal direction of the humidifying chamber 45 of the three water adsorbing filter elements 42 sequentially increase in the arrangement direction. As shown in FIG. 10, after the three water adsorbing filter elements 42 are installed within the installation region of the water adsorbing filter elements, when the humidifying liquid in the humidifying chamber 45 is at the normal-operating-state water level, the depths of the soaking of the water adsorbing filter elements 42 into the humidifying liquid inside the humidifying chamber 45 are unequal, and, according to the principle of capillary effect, the humidifying liquid climbs along the water absorbing fiber till the water absorbing fiber fully contains water. The three third monitors 91 are located behind the three water adsorbing filter elements 42, and are used for monitoring the gas property (for example, temperature and/or humidity) after the gas passes through the water adsorbing filter elements 42. When the ventilation-treatment device is in the normal treatment state, the gas, after entering the humidifying chamber 45 via the gas inlet 43 of the housing 41, sequentially passes through the three water adsorbing filter elements 42, and brings the warm and wet gas to the patient side via the gas outlet 44 for the patient to use, at which point all of the three water adsorbing filter elements 42 fully contain the humidifying liquid, and all of the three third monitors 91 collect the gas property of the normal operating state.

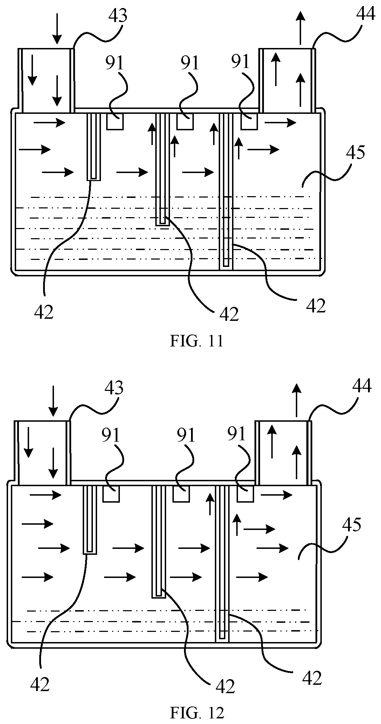

As shown in FIG. 11, when the humidifying liquid in the humidifying chamber 45 is lower than the height of the first water adsorbing filter element 42 (assuming that its height is at the position of a half of the humidifying chamber 45), the first water adsorbing filter element 42 has disengaged from the surface of the humidifying liquid, the last two water adsorbing filter elements 42 still fully contain the humidifying liquid, and the gas property monitored by the first third monitor 91 is different from the gas properties monitored by the last two third monitors 91.

As shown in FIG. 12, when the humidifying liquid in the humidifying chamber 45 is lower than the height of the second water adsorbing filter element 42 (assuming that its height is at the position of a quarter of the humidifying chamber 45), the first and the second water adsorbing filter elements 42 have disengaged from the surface of the humidifying liquid, the first water adsorbing filter element 42 still fully contains the humidifying liquid, and the gas properties monitored by the first and the second third monitors 91 are different from the gas property monitored by the third third monitor 91.

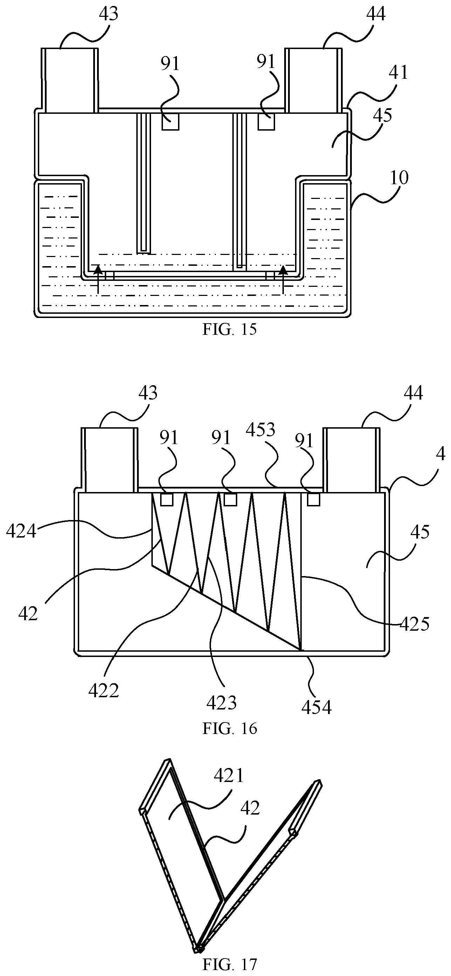

In an embodiment of the present disclosure, the water adsorbing filter elements 42 may also incline. Optionally, in the embodiments shown in FIGs. 16-25, at least some of the water adsorbing filter elements 42 incline relatively to the transverse direction of the humidifying chamber 45, and extend in the inclined direction from the top to the bottom of the humidifying chamber 45 to form the first cross section 421.

In this case, by inclining some of the water adsorbing filter elements 42, the contact area with the gas flow may be increased, and the gas flow may be intercepted and humidified from different angles, which, by the humidification of multiple layers and different angles, may effectively improve the effect of humidification.

As an alternative embodiment, as shown in FIG. 16, the water adsorbing filter elements 42 inclining relatively to the transverse direction of the humidifying chamber 45 include a plurality of first water adsorbing filter elements 422 and a plurality of second water adsorbing filter elements 423 that are arranged alternately inside the humidifying chamber 45 in the gas-flow direction, and the directions of the inclining relative to the transverse direction of the humidifying chamber 45 of the first water adsorbing filter elements 422 and the second water adsorbing filter elements 423 are opposite.

Here, the plurality of first water adsorbing filter elements 422 and the plurality of second water adsorbing filter elements 423 that are alternately arranged and incline in opposite directions may be provided inside the humidifying chamber 45. After the external gas enters the humidifying chamber 45, it sequentially passes through the multiple layers of the first water adsorbing filter elements 422 and the second water adsorbing filter elements 423 that are alternately arranged, and the layers of the first water adsorbing filter elements 422 and the second water adsorbing filter elements 423 may intercept and humidify the gas flow from different angles, which, by the humidification of multiple layers and different angles, may effectively improve the effect of humidification.

Preferably, as shown in FIGs. 16 and 17, neighboring first water adsorbing filter elements 422 and second water adsorbing filter elements 423 form a V shape therebetween.

In this case, the plurality of groups of first water adsorbing filter elements 422 and second water adsorbing filter elements 423 that form the V shape are sequentially arranged inside the humidifying chamber 45. After the external gas enters the humidifying chamber 45, the V-shaped first water adsorbing filter elements 422 and second water adsorbing filter elements 423 may intercept and humidify the gas flow from different angles, to realize multilayer humidification and multi-angular humidification, which may effectively improve the effect of humidification. Furthermore, the V-shaped arrangement is more aesthetically attractive, and is more convenient to manufacture and assemble.

As an alternative embodiment, a water adsorbing filter element 424 arranged at the first position in the gas-flow direction and a water adsorbing filter element 425 arranged at the last position in the gas-flow direction individually extend in the direction from the top to the bottom of the humidifying chamber 45 to form the first cross section 421; and

the first water adsorbing filter elements 422 and the second water adsorbing filter elements 423 are arranged alternately between the water adsorbing filter element 424 disposed at the first position and the water adsorbing filter element 425 disposed at the last position.

In this case, the water adsorbing filter element 424 disposed at the first position and the water adsorbing filter element 425 disposed at the last position extend in the direction from the top to the bottom of the humidifying chamber 45, and the formed first cross section 421 intersects perpendicularly with the gas-flow direction. The first cross sections 421 of the two water adsorbing filter elements completely penetrate a second cross section inside the humidifying chamber 45 between the gas inlet 43 and the gas outlet 44 in the orthogonal direction. The gas flow, when passing through the two water adsorbing filter elements, may contact the entire surface of the two water adsorbing filter elements, which increases the contact area, may bring away more humidifying liquid, and improves the effect of humidification.

Furthermore, the plurality of first water adsorbing filter elements 422 and the plurality of second water adsorbing filter elements 423 are arranged alternately between the water adsorbing filter element 424 disposed at the first position and the water adsorbing filter element 425 disposed at the last position. Accordingly, after the gas flow enters the humidifying chamber 45, it may sequentially pass through the water adsorbing filter element 424 disposed at the first position, the plurality of first water adsorbing filter elements 422 and the plurality of second water adsorbing filter elements 423, and the water adsorbing filter element 425 disposed at the last position, to realize multilayer humidification and multi-angular humidification, to effectively improve the effect of humidification.

In an embodiment of the present disclosure, the water-absorbing-fiber device including the multiple layers of the water adsorbing filter elements 42 may be configured to be of an integrated structure, to facilitate the installation and the replacement.

As stated above, each of the different water adsorbing filter elements 42 may be provided with one third monitor 91 downstream, to monitor the downstream gas property. When the gas property monitored by any one of the third monitors 91 is abnormal, a state of liquid insufficiency may be detected in time, to achieve the purpose of preventing dry heating.

For example, the third monitors 91 may be provided downstream of the water adsorbing filter element 42 close to the gas inlet 43 and downstream of the water adsorbing filter element 42 close to the gas outlet 44. In order to control in detail the amount of the humidifying liquid inside the humidifier 4, a third monitor may also be provided downstream of the water adsorbing filter element 42 at a midstream position. When the amount of the humidifying liquid gradually decreases, the gas properties collected by each of the third monitors 91 change. When the gas property monitored by any one of the third monitors 91 is abnormal, a state of liquid insufficiency may be detected in time, and a corresponding controlling strategy may be made, to achieve the purpose of preventing dry heating.

In this case, by providing the plurality of third monitors 91 for multilayer monitoring, the safety is improved.

The gas property monitored by the third monitors 91 may be the temperature and/or the humidity of the gas. When the gas entering the humidifier 4 is a heated dry air, that has a better effect for the humidification.

As an alternative embodiment, each two neighboring first water adsorbing filter element 422 and the second water adsorbing filter element 422 are grouped as one group, and each of the remaining individual water adsorbing filter elements 42 is grouped as one group; and
the lengths of the extension in the direction from the top to the bottom of the humidifying chamber 45 of different groups of the water adsorbing filter elements 42 are unequal.

In this case, as shown in FIG. 16, each two neighboring first water adsorbing filter elements 422 and second water adsorbing filter elements 423, i.e., two water adsorbing filter elements 42 that form a V shape, may be grouped as one group, and each of the remaining individual water adsorbing filter elements 42 is grouped as one group. The lengths of the extension in the direction from the top to the bottom of the humidifying chamber 45 of different groups of the water adsorbing filter elements 42 are unequal. furthermore, the different water adsorbing filter elements 42 are provided with the third monitors 91 downstream. Liquid lacking of different degrees inside the humidifying chamber 45 may be monitored, to control the humidifier 4 according thereto, to prevent dry heating.

The remaining individual water adsorbing filter elements 42 include the water adsorbing filter element 424 disposed at the first position and the water adsorbing filter element 425 disposed at the last position. If finally one single first water adsorbing filter element 422 is left, and has no pairing second water adsorbing filter element 423, then the remaining individual water adsorbing filter elements 42 further include the one single first water adsorbing filter element 422. In this case, the water adsorbing filter element 424 disposed at the first position may be grouped as one group, the water adsorbing filter element 425 disposed at the last position may be grouped as one group, and the one single first water adsorbing filter element 422 that is finally left may also be grouped as one group, wherein the extension lengths of them are unequal to those of the other groups of the water adsorbing filter elements, so as to achieve the purpose of monitoring liquid lacking of different degrees inside the humidifying chamber 45.

Optionally, it may be configured that the lengths of the extension in the direction from the top to the bottom of the humidifying chamber 45 of different groups of the water adsorbing filter elements 42 decrease or increase sequentially in the arrangement direction.

In this case, by providing, inside the humidifying chamber 45, the plurality of groups of the water adsorbing filter elements 42 whose lengths sequentially decrease or increase and that have the gradually changing gradients, and providing one third monitor 91 downstream of each of the different water adsorbing filter elements 42, liquid lacking of different degrees inside the humidifying chamber 45 may be monitored, to control the humidifier 4 according thereto, to prevent dry heating.

For example, in the embodiments shown in FIGs. 16-19, seven groups of the water adsorbing filter elements 42 may be provided inside the humidifying chamber 45. The first group is the water adsorbing filter element 424 disposed at the first position that does not incline, the second to the five groups are the V-shaped water adsorbing filter elements 42 arranged in the middle, the sixth group is the last one inclined first water adsorbing filter element 422, and the seventh group is the water adsorbing filter element 424 disposed at the last position that does not incline. The seven groups of water adsorbing filter elements 42 are sequentially arranged in the gas-flow direction, and are located between the gas inlet 43 and the gas outlet 44, and the lengths of the extension from the top to the bottom of the humidifying chamber 45 of the seven groups of water adsorbing filter elements 42 sequentially increase in the arrangement direction. The seven groups of water adsorbing filter elements 42 may be configured to be of an integrated structure, to facilitate the detaching and the installation. Furthermore, one third monitor 91 may be provided downstream of each of the second group of the water adsorbing filter elements 42, the fourth group of the water adsorbing filter elements 42 and the seventh group of the water adsorbing filter elements 42.

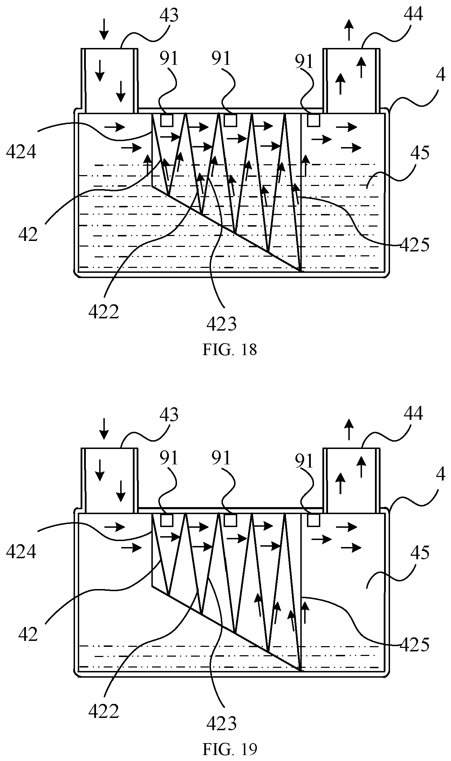

As shown in FIG. 18, after the water-absorbing-fiber device including the seven groups of water adsorbing filter elements 42 is installed within the corresponding installation region, when the humidifying liquid in the humidifying chamber 45 is at the normal-operating-state water level, the depths of the soaking of the seven groups of water adsorbing filter elements 42 into the humidifying liquid inside the humidifying chamber 45 are unequal, and, according to the principle of capillary effect, the humidifying liquid climbs along the water absorbing fiber till the water absorbing fiber fully contains water. Three third monitors 91 are located behind three of the water adsorbing filter elements 42, and are used for monitoring the gas property (for example, temperature and/or humidity) after the gas passes through the water adsorbing filter elements 42. When the ventilation-treatment device is in the normal treatment state, the gas, after entering the humidifying chamber 45 via the gas inlet 43 of the housing 41, sequentially passes through the seven groups of water adsorbing filter elements 42, and brings the warm and wet gas to the patient side via the gas outlet 44 for the patient to use, at which point all of the seven groups of water adsorbing filter elements 42 fully contain the humidifying liquid, and all of the three third monitors 91 collect the gas property of the normal operating state.

As shown in FIG. 19, when the humidifying liquid in the humidifying chamber 45 is insufficient or lacked, the water adsorbing filter element 42 close to the gas outlet is closer to the liquid surface, the fifth group of the V-shaped water adsorbing filter elements 42, the sixth group of the inclining first water adsorbing filter element 422 and the water adsorbing filter element 425 disposed at the last position still fully contain the humidifying liquid, and the first several groups of the water adsorbing filter elements 42 have already disengaged from the liquid surface, in which the water absorbing fiber contains no or little humidifying liquid. At this point, the gas entering via the gas inlet 43 may merely bring away the humidifying liquid from the last several groups of the water adsorbing filter elements 42, and cannot bring away the humidifying liquid or brings away a small amount of the humidifying liquid from the first several groups of the water adsorbing filter elements 42. Therefore, the gas property collected by the last one third monitor 91 is substantially the same as that of the normal operating state, while, in the gas properties collected by the first and the second third monitors 91, as compared with the normal operating state, the temperature increases and the humidity decreases. The three third monitors 91 may send the collected gas properties to the controlling system, and the controlling system reduces the heating power of the humidifier 4 or stops the heating of the humidifier 4, to prevent dry heating of the humidifier 4.

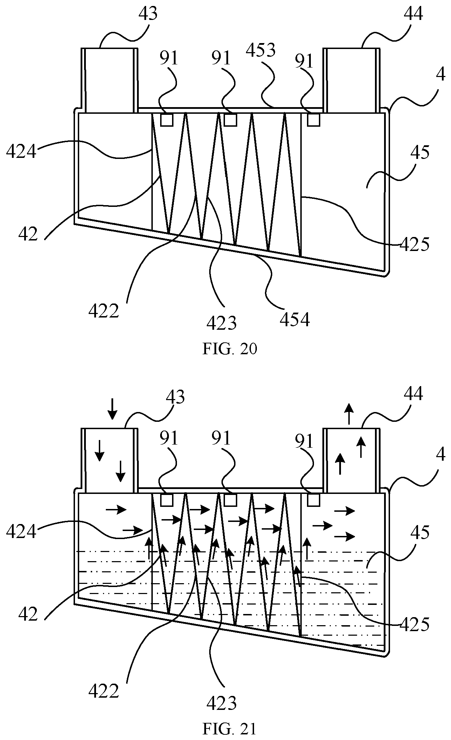

As another alternative embodiment, referring to FIGs. 20-22, the bottom of the humidifying chamber 45 inclines, and each of the water adsorbing filter elements 42 extends in the extension direction from the top to the bottom of the humidifying chamber 45.

In this case, by configuring the bottom of the humidifying chamber 45 to be inclined, and causing each of the water adsorbing filter elements 42 to extend from the top to the bottom of the humidifying chamber 45, the plurality of groups of the water adsorbing filter elements 42 whose lengths sequentially decrease or increase and that have the gradually changing gradients may also be obtained. Furthermore, by providing one third monitor 91 downstream of each of the different water adsorbing filter elements 42, liquid lacking of different degrees inside the humidifying chamber 45 may be monitored, to control the humidifier 4 according thereto, to prevent dry heating.

For example, in the embodiments shown in FIGs. 20-22, the bottom of the humidifying chamber 45 may incline, and seven groups of the water adsorbing filter elements 42 may be provided inside the humidifying chamber 45. The first group is the water adsorbing filter element 424 disposed at the first position that does not incline, the second to the five groups are the V-shaped water adsorbing filter elements 42 arranged in the middle, the sixth group is the last one inclined first water adsorbing filter element 422, and the seventh group is the water adsorbing filter element 424 disposed at the last position that does not incline. The seven groups of water adsorbing filter elements 42 are sequentially arranged in the gas-flow direction, and are located between the gas inlet 43 and the gas outlet 44, and the seven groups of water adsorbing filter elements 42 extend in the extension direction from the top to the bottom of the humidifying chamber 45. The extension lengths of the seven groups of water adsorbing filter elements 42 that are accordingly obtained sequentially increase in the arrangement direction. The seven groups of water adsorbing filter elements 42 may be configured to be of an integrated structure, to facilitate the detaching and the installation. Furthermore, one third monitor 91 may be provided downstream of each of the second group of the water adsorbing filter elements 42, the fourth group of the water adsorbing filter elements 42 and the seventh group of the water adsorbing filter elements 42.

As shown in FIG. 21, after the water-absorbing-fiber device including the seven groups of water adsorbing filter elements 42 is installed within the corresponding installation region, when the humidifying liquid in the humidifying chamber 45 is at the normal-operating-state water level, the depths of the soaking of the seven groups of water adsorbing filter elements 42 into the humidifying liquid inside the humidifying chamber 45 are unequal, and, according to the principle of capillary effect, the humidifying liquid climbs along the water absorbing fiber till the water absorbing fiber fully contains water. Three third monitors 91 are located behind three of the water adsorbing filter elements 42, respectively, and are used for monitoring the gas property (for example, temperature and/or humidity) after the gas passes through the water adsorbing filter elements 42. When the ventilation-treatment device is in the normal treatment state, the gas, after entering the humidifying chamber 45 via the gas inlet 43 of the housing 41, sequentially passes through the seven groups of water adsorbing filter elements 42, and brings the warm and wet gas to the patient side via the gas outlet 44 for the patient to use, at which point all of the seven groups of water adsorbing filter elements 42 fully contain the humidifying liquid, and all of the three third monitors 91 collect the gas property of the normal operating state.

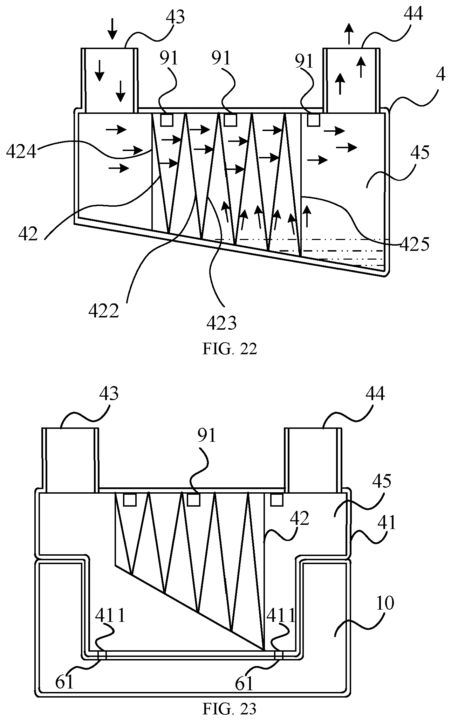

As shown in FIG. 22, when the humidifying liquid in the humidifying chamber 45 is insufficient or lacked, the water adsorbing filter element 42 close to the gas outlet is closer to the liquid surface, the fourth group and the fifth group of the V-shaped water adsorbing filter elements 42, the sixth group of the inclining first water adsorbing filter element 422 and the water adsorbing filter element 425 disposed at the last position still fully contain the humidifying liquid, and the first several groups of the water adsorbing filter elements 42 have already disengaged from the liquid surface, in which the water absorbing fiber contains no or little humidifying liquid. At this point, the gas entering via the gas inlet 43 may merely bring away the humidifying liquid from the last several groups of the water adsorbing filter elements 42, and cannot bring away the humidifying liquid or brings away a small amount of the humidifying liquid from the first several groups of the water adsorbing filter elements 42. Therefore, the gas properties collected by the second and the last third monitors 91 are substantially the same as that of the normal operating state, while, in the gas property collected by the first third monitor 91, as compared with the normal operating state, the temperature increases and the humidity decreases. The three third monitors 91 may send the collected gas properties to the controlling system, and the controlling system reduces the heating power of the humidifier 4 or stops the heating of the humidifier 4, to prevent dry heating of the humidifier 4.

Further, in the embodiment in which the bottom of the humidifying chamber 45 inclines and each of the water adsorbing filter elements 42 extends in the extension direction from the top to the bottom of the humidifying chamber 45, each of the water adsorbing filter elements 42 may be connected to the top and the bottom of the humidifying chamber 45, to ensure that each of the water adsorbing filter elements 42 may contact the humidifying liquid to the largest extent, thereby prolonging the humidification duration of the humidifier 4, and enabling the water adsorbing filter elements 42 to be more stable.

In an embodiment of the present disclosure, the third monitors 91, after collecting the gas property, may emit an instruction to a controlling system, to reduce the heating power of the humidifier 4 or stop the heating of the humidifier 4, to prevent dry heating of the humidifier 4. The controlling system may be provided at the side of the humidifier 4, and may also be provided at the side of the ventilation-treatment device. When it is provided at the side of the humidifier 4, as an alternative embodiment, the humidifier 4 further includes:
a first controller, wherein the first controller is electrically connected to the monitoring device 9, and is for, based on the gas property monitored by the monitoring device 9, controlling the operation state of the humidifier 4, and/or, the operation state of a ventilation-treatment-device mainframe in communication with the humidifier 4.

In this case, the first controller may be provided inside the humidifier 4, and the first controller, based on the monitoring result of the monitoring device 9, reasonably controls the operation state of the humidifier 4 or the operation state of the ventilation-treatment-device mainframe, to prevent dry heating. For example, when the monitoring result of the monitoring device 9 indicates that the interior of the humidifier 4 seriously lacks the humidifying liquid, the heating function of the humidifier 4 is stopped or the operating state of the ventilation-treatment device is stopped.

The first controller may provide the controlling strategy based on the mode of the provision of the water adsorbing filter elements 42. For example, if two or more water adsorbing filter elements 42 of unequal lengths are provided inside the humidifying chamber 45 of the humidifier 4, then the first controller may correspondingly provide two or more layers of controlling strategy. When the residual amount of the humidifying liquid inside the humidifying chamber 45 of the humidifier 4 is lower than a first water level, wherein the first water level represents that the remaining humidifying liquid may still support the humidifier 4 to continuously operate, but has already been consumed partially, then at this point the third monitor 91 behind the shortest or medium-length water adsorbing filter element 42 monitors a gas-property abnormality (marked as a first abnormal state). The first controller monitors the first abnormal state, and may, based on the current heating power, reduce the heating power of the humidifier 4, to prolong the usage duration of the ventilation-treatment device, to prevent the humidifying liquid from being quickly consumed off. When the residual amount of the humidifying liquid inside the humidifying chamber 45 of the humidifier 4 is lower than a second water level, the second water level represents that the remaining humidifying liquid has already been insufficient to support the humidifier 4 to continuously operate, or even there has already been no humidifying liquid, then at this point the third monitor 91 behind the longest water adsorbing filter element 42 monitors the gas-property abnormality (marked as a second abnormal state). The first controller monitors the second abnormal state, and may shut down the heating function of the humidifier 4 or stop the operating state of the ventilation-treatment-device mainframe, to prevent dry heating.

A particular example of the application will be provided below:

When the patient requires wearing the ventilation-treatment device (for example, a breathing machine) for a long time, it is very important that a warm and wet gas flow may be continuously inputted. Otherwise, because the humidifying liquid is insufficient, in the first half period of the machine wearing, the warm and wet gas flow is inputted, and in the second half period, a dry and hot gas flow is inputted due to water insufficiency, which results in very poor comfortableness and experience of the patient. In an embodiment of the present disclosure, a three-layer water amount monitoring function may be provided, that is, three water adsorbing filter elements 42 whose lengths sequentially increase are provided inside the humidifying chamber 45, and one third monitor 91 is provided behind each of the water adsorbing filter elements 42.

It is assumed that the patient continuously uses the device for 8 hours. If the machine wearing reaches 4 hours, the residual amount of the humidifying liquid is lower than the height of the first water adsorbing filter element 42 that is the shortest (assuming that its height is at the position of a half of the humidifying chamber 45), the first third monitor 91 monitors the gas-property abnormality, and emits an instruction to the first controller, and the first controller suitably adjusts downwardly the humidification level, to satisfy a uniform outputting of the warm and wet gas flow during the whole process of the machine wearing.

If the machine wearing reaches 6 hours, the residual amount of the humidifying liquid is lower than the height of the second water adsorbing filter element 42 of the medium length (assuming that its height is at the position of a quarter of the humidifying chamber 45), the first and the second third monitors 91 monitor the gas-property abnormality, and emit an instruction to the first controller, and the first controller further adjusts downwardly the humidification level, to satisfy outputting of the warm and wet gas flow during the remaining process of the machine wearing.

If the machine wearing reaches 8 hours, the humidifying liquid is lower than the height of the first water adsorbing filter element 42 that is the longest, all of three third monitors 91 monitor the gas-property abnormality, and emit an instruction to the first controller, and the first controller shuts down the heating function of the humidifier 4 or stops the operating state of the breathing machine, to prevent dry heating, to prevent aging of the heating region and its adjacent sealing components of the humidifier 4 due to long-time high temperature, and uncomfortableness or even the risk of patient ambustion due to the dry and hot gas flow.

Certainly, the above-described controlling strategy is merely an example for the description. In the embodiments of the present disclosure, in practical applications, any plurality of or plurality of groups of the water adsorbing filter elements 42 of unequal lengths may be provided, and accordingly different controlling strategies of the humidifier 4 may be made, which may have various variations, and is not described in detail herein.

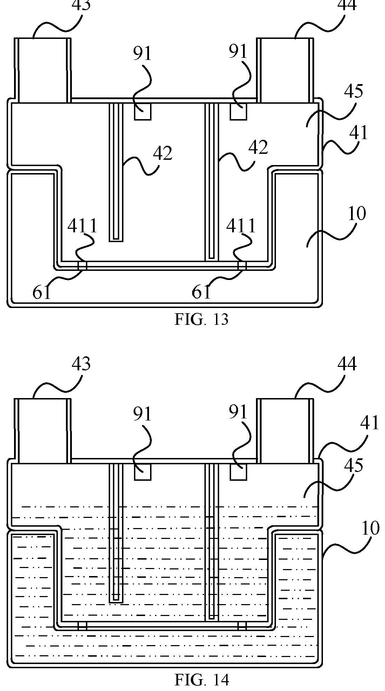

In order to further prevent dry heating, and prolong the usage duration of the ventilation-treatment device, as shown in FIGs. 13 and 23, optionally, the humidifier 4 further includes:
a standby water tank 10, wherein the standby water tank 10 is provided at the bottom of the housing 41, a first communicating component is provided at the top of the standby water tank 10, a second communicating component in cooperation with the first communicating component is provided at the bottom of the housing 41, and the housing 41 and the standby water tank 10 are communicated by the cooperation between the first communicating component and second communicating component.

In this case, by providing the standby water tank 10 at the bottom of the housing 41, when the humidifying liquid inside the humidifying chamber 45 of the housing 41 is insufficient, the standby water tank 10 may supplement the liquid to the humidifying chamber 45, to further prevent dry heating, and prolong the usage duration of the ventilation-treatment device.

Optionally, as shown in FIGs. 13 and 23, the first communicating component includes at least three first communicating holes 61 that are evenly distributed, and the second communicating component includes at least three second communicating holes 411 that cooperate with the first communicating holes. The first communicating holes 61 and the second communicating holes 411 may be sealed by using a sealing material at the periphery, to prevent water leakage.

In this case, the housing 41 and the standby water tank 10 are communicated by the correspondingly arranged communicating holes, and the communicating holes are sealed at the periphery. Three or more communicating holes that are evenly distributed are provided at the periphery of the outer side of the housing 41, which may ensure that, when inclined, the housing 41 may maintain the communication with the standby water tank 10 in all directions, to prevent warping at a certain position in inclining, which affects the effect of the communication.

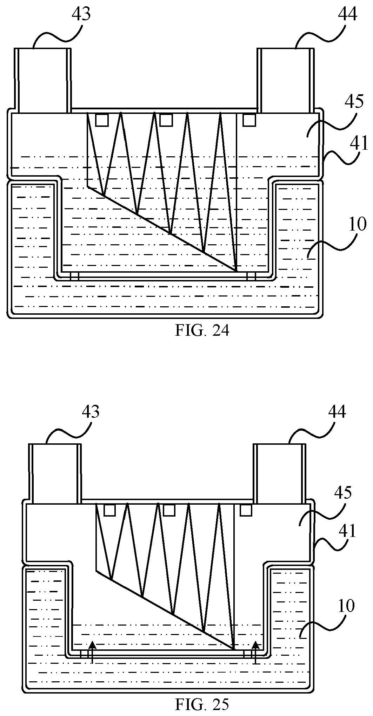

As shown in FIGs. 14 and 24, when the humidifying liquid inside the humidifying chamber 45 of the housing 41 is at the normal-operating-state water level, the standby water tank 10 is full of water, the water level in the humidifying chamber 45 of the housing 41 is higher than the standby water tank 10, and the ventilation-treatment device (for example, a breathing machine) and the humidifier 4 normally operate.

As shown in FIGs. 15 and 25, when the water level of the humidifying liquid inside the humidifying chamber 45 of the housing 41 is lower than the water level on the outer side of the standby water tank 10, the humidifying liquid on the outer side of the standby water tank 10 is supplemented to the humidifying chamber 45 of the housing 41 via the communicating holes, to maintain the long-time and continuous usage of the device by the patient.

As an alternative embodiment, self-sealing silica-gel layers are provided on the first communicating holes 61, and hollow cylinders that may pass through the self-sealing silica-gel layers are provided under the second communicating holes 411.

In this case, the communicating holes of the standby water tank 10 are sealed by the self-sealing silica-gel layers, and the liquid therein cannot be leaked in normal storage and transportation. The hollow cylinders are provided under the communicating holes of the housing 41, and when it is not required to use the standby water tank 10 in a short time, the hollow cylinders under the communicating holes of the housing 41 may be closed by using silica-gel plugs for usage. When long-time and continuous usage requires externally connecting the standby water tank 10, the silica-gel plugs are taken down, the housing 41 is installed to the standby water tank 10 with the communicating holes matched, and the hollow cylinders pass through the self-sealing silica-gel layers of the communicating holes of the standby water tank 10, to communicate the housing 41 and the standby water tank 10.

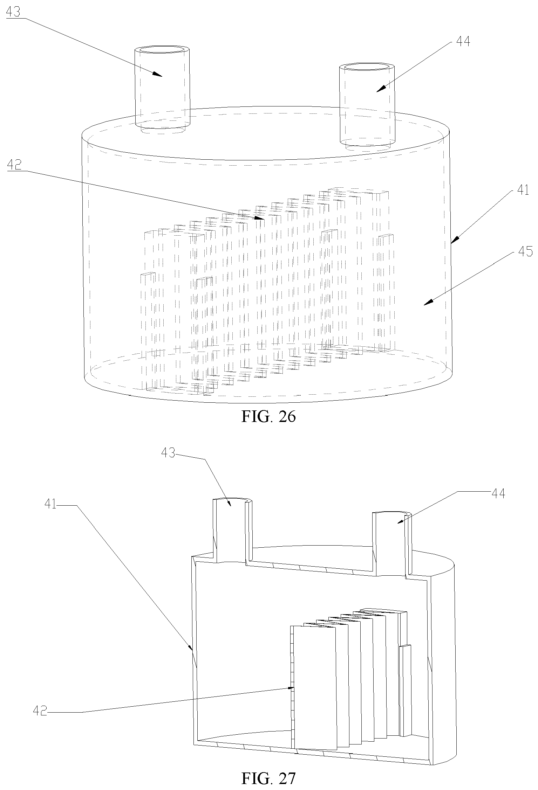

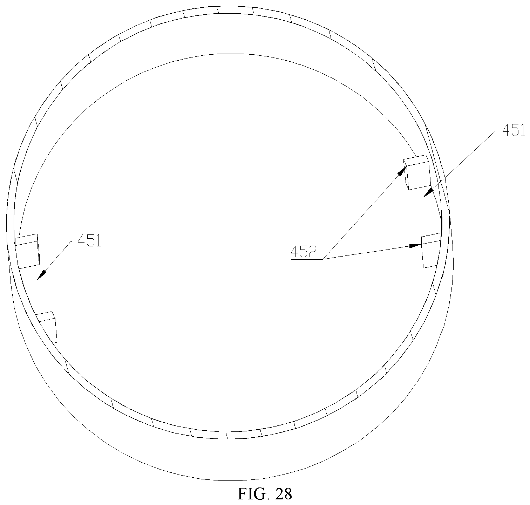

Referring to FIGs. 26-29, FIGs. 26-29 show another humidifier 4 according to the embodiments of the present disclosure. The humidifier 4 includes a housing 41. The housing 41 defines a humidifying chamber 45. The humidifying chamber 45 is used for containing the humidifying liquid. The humidifier 4 further includes water adsorbing filter elements 42 provided in the humidifying chamber 45. The lower ends of the water adsorbing filter elements 42 are soaked into the humidifying liquid inside the humidifying chamber 45.

Accordingly, because of the capillary effect, the humidifying liquid climbs along the water adsorbing filter elements 42, whereby the whole of the water adsorbing filter elements 42 may adsorb the humidifying liquid, which increases the humidification area in the humidifying chamber 45. The gas entering the humidifying chamber 45, besides being humidified at the liquid level of the humidifying liquid, may also be humidified when passing through the water adsorbing filter elements 42 fully containing water, thereby obtaining a more excellent humidification effect of a higher efficiency, which facilitates miniaturized ventilation-treatment devices to obtain an excellent humidification rate, optimizes the user experience, and has high safety and reliability.

In order to ensure that the bottoms of the water adsorbing filter elements 42 may always be soaked in the humidifying liquid, it may configure that the bottoms of the water adsorbing filter elements 42 support the bottom of the humidifying chamber 45.

Regarding the humidifier 4, a gas inlet 43 and a gas outlet 44 are formed in the housing 41. The gas enters the humidifying chamber 45 via the gas inlet 43, is humidified in the humidifying chamber 45, and subsequently is discharged via the gas outlet 44. In other words, the flowing direction of the gas flow in the humidifier 4 is generally the direction from the gas inlet 43 to the gas outlet 44. Preferably, the gas inlet 43 and the gas outlet 44 are located on two sides of the water adsorbing filter elements 42, so that the whole of the gas flow entering the humidifying chamber 45 is discharged after passing through the water adsorbing filter elements 42 to the largest extent, thereby obtaining a more excellent humidification effect of a higher efficiency.

Regarding the mode of the installation of the water adsorbing filter elements 42 inside the humidifying chamber 45, alternatively, two water-adsorbing-filter-element installing units 451 are formed on the inner side wall of the humidifying chamber 45, and the two ends of the water adsorbing filter elements in the extension direction are installed to the water-adsorbing-filter-element installing units 451 on the corresponding sides.

Optionally, the two water-adsorbing-filter-element installing units 451 are opposite to each other. Accordingly, the water adsorbing filter elements 42 are formed in the form similar to filtering screens, which extend throughout a whole first direction of the humidifying chamber 45, wherein the first direction has an included angle with (for example, perpendicular to) the direction from the gas inlet 43 to the gas outlet 44. All of the gases flowing from the gas inlet 43 to the gas outlet 44 need to pass through the first direction; in other words, the gases flow into the water adsorbing filter elements 42 to the largest extent, thereby obtaining a more excellent humidification effect of a higher efficiency.

Particularly, in the illustrated embodiments, the humidifying chamber 45 is generally of a cylindrical shape, the water adsorbing filter elements 42 extend in a certain radial direction (the first direction) of the humidifying chamber 45, and the connecting line between the centers of the gas inlet 43 and the gas outlet 44 is another radial direction of the humidifying chamber 45, and the another radial direction is perpendicular to the first direction.

Particularly, each of the water-adsorbing-filter-element installing units 451 includes two parallel vertical blocking plates 452 formed by protruding out of the inner side wall of the humidifying chamber 45, and a socket defined between the two parallel vertical blocking plates 452. The two ends of the water adsorbing filter elements 42 may be inserted into the sockets on the corresponding sides, which may conveniently realize the detaching of the water adsorbing filter elements 42 from the humidifying chamber 45. To configure the water adsorbing filter elements 42 to be detachable facilitates replacement when necessary, and periodical replacement facilitates to guarantee an excellent and high-efficiency humidification effect.

Furthermore, regarding the water adsorbing filter elements 42 themselves, they may be paper or a fiber fabric; for example, they may be non-woven fabrics. Preferably, they have a certain hardness or toughness, and are folded in the humidifying chamber 45, to obtain a higher humidification area. Furthermore, the water adsorbing filter elements 42 themselves may be manufactured by using a fiber material having a gas permeability. Certainly, alternatively, the water adsorbing filter elements 42 may be provided with some small through holes, for the gas flow to pass through. In addition, the water adsorbing filter elements 42, besides the paper or fiber fabric, may further include hard sides encircling the periphery of the paper or fiber fabric, to facilitate to shape the water adsorbing filter elements 42 and/or facilitate to insert them into the sockets.

Regarding the mode of the folding of the water adsorbing filter elements 42, as an embodiment, the water adsorbing filter elements 42 include folded water adsorbing filtering discs, and the central plane of the water adsorbing filtering discs, when cut in the extension direction, obtains a folded line. It should be noted that the "central plane of the water adsorbing filter elements 42" described herein refers to the plane that is formed by connecting the center points in the thickness direction of the positions of the water adsorbing filter elements 42.

For example, the water adsorbing filtering discs are folded as a V shape, and the folding angle of the folded line is less than 80°, preferably 15°-30°, to obtain a higher humidification area, to optimize the effect of humidification.

Moreover, the height of the water adsorbing filter elements 42 is required to be higher than the liquid level, and, according to demands, may form a certain gap with the top wall of the humidifying chamber 45, to prevent influencing the gas resistance. Preferably, the flowing area at the gap is greater than or equal to the smallest flowing area when the gas passes through all of the paths in the device, and the gap may be small to the greatest extent on the precondition that the gas resistance is not influenced. Furthermore, the tops of the water adsorbing filter elements 42 may have equal heights (flushed), and may also have irregular shapes and thus be staggered in the heights.

In the humidifier 4 according to the embodiments of the present disclosure, by providing the component that may monitor the water level inside the humidifier 4, dry heating of the humidifier 4 is prevented, and when it is monitored that the humidifier 4 lacks the humidifying liquid, the controlling system of the monitoring device 9 stops the operating state of the breathing machine or stops the heating function of the humidifier 4, thereby preventing injuring the patient and damaging the device.

In the humidifier 4 according to the embodiments of the present disclosure, the monitoring device 9 monitors the residual amount of the humidifying liquid in real time, and the patient or the user is not required to always pay attention, which improves the convenience. Furthermore, dry heating may be prevented, whereby the heating region and the adjacent sealing components of the humidifier are not continuously in a high-temperature environment, and thus do not easily age, which prolongs the service life of the product. The device has a humanized design, and has high safety and reliability.

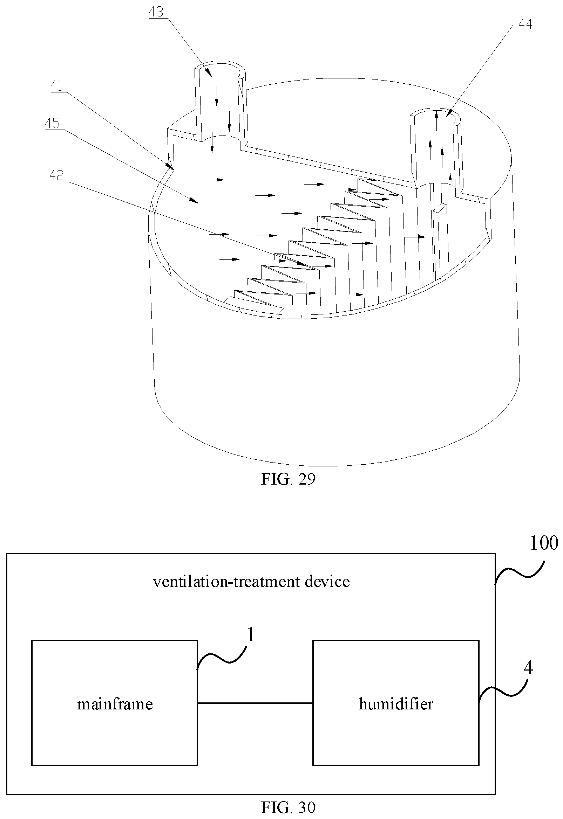

Referring to FIG. 30, an embodiment of the present disclosure further provides a ventilation-treatment device 100. The ventilation-treatment device includes a mainframe 1 for generating a gas of a preset pressure, and the mainframe 1 includes a gas outlet channel. The ventilation-treatment device 100 further includes: the humidifier 4 stated above, and the gas outlet channel of the mainframe 1 is in communication with the gas inlet of the humidifier 4.

When the ventilation-treatment device 100 according to the embodiments of the present disclosure is being used, the gas outlet channel of the mainframe 1 may be communicated with the gas inlet 43 of the humidifier. After the humidifying liquid (for example, water) is placed inside the humidifying chamber and the humidifying liquid contacts the water adsorbing filter elements 42 of the water-absorbing-fiber device, because of the capillary effect, the humidifying liquid climbs along the water adsorbing filter elements 42, whereby the water adsorbing filter elements 42 fully contain the humidifying liquid. The first cross section 421 of the water adsorbing filter elements 42 intersects with the gas-flow direction. When the ventilation-treatment device is in the normal ventilation-treatment state, the gas enters the humidifying chamber 45 of the housing 41 via the gas inlet 43, sequentially passes through the plurality of sequentially arranged water adsorbing filter elements 42, contacts the water adsorbing filter elements 42, and brings away the humidifying liquid in the water adsorbing filter elements 42, to reach the effect of humidification. When the liquid inside the humidifying chamber 45 is insufficient, the water adsorbing filter elements 42 of the water-absorbing-fiber device lack the humidifying liquid, the gas cannot reach the effect of humidification when passing through the water-absorbing-fiber device, and the gas downstream of the water-absorbing-fiber device, as compared with the state not lacking the humidifying liquid, has an increased temperature and a reduced humidity. Therefore, by monitoring the gas property downstream of the water-absorbing-fiber device by using the monitoring device 9, a state of liquid insufficiency may be detected in time, to achieve the purpose of preventing dry heating. Accordingly, by providing the water-absorbing-fiber device and the monitoring device 9, dry heating of the humidifier may be effectively prevented without the patient or the user always paying attention to the residual amount of the humidifying liquid, which prevents component damage and patient ambustion caused by dry heating of the humidifier 4, thereby prolonging the service life of the product, and improving the safety and the reliability.

Optionally, the ventilation-treatment device 100 further includes:
a second controller, wherein the second controller is electrically connected to the monitoring device, is electrically connected to the mainframe 1 and/or the humidifier 4, and is for, based on the gas property monitored by the monitoring device, controlling the operation state of the mainframe 1 and/or the humidifier 4.

In this case, the second controller may be provided inside the ventilation-treatment device 100, and the second controller, based on the monitoring result of the monitoring device, reasonably controls the operation state of the mainframe 1 or the humidifier 4, to prevent dry heating.

Particularly, the second controller may, when the monitored gas property is in the first abnormal state, reduce the heating power of the humidifier 4, and/or, when the gas property is in the second abnormal state, shut down the heating function of the humidifier 4 or stop the operating state of the ventilation-treatment-device mainframe 1.

In this case, when the monitored gas property is in the first abnormal state, wherein the first abnormal state indicates that the residual amount of the humidifying liquid inside the humidifying chamber of the humidifier 4 is lower than a first water level, wherein the first water level represents that the remaining humidifying liquid may still support the humidifier 4 to continuously operate, but has already been consumed partially, then the heating power of the humidifier 4 is reduced, to prolong the usage duration of the ventilation-treatment device, to prevent the humidifying liquid from being quickly consumed off. When the monitored gas property is in the second abnormal state, wherein the second abnormal state indicates that the residual amount of the humidifying liquid inside the humidifying chamber of the humidifier 4 is lower than a second water level, wherein the second water level represents that the remaining humidifying liquid has already been insufficient to support the humidifier 4 to continuously operate, or even there has already been no humidifying liquid, then the heating function of the humidifier 4 is shut down or the operating state of the ventilation-treatment-device mainframe 1 is stopped, to prevent dry heating.

What type of the abnormal states the gas property is in may refer to the above description on the embodiments of the humidifier side, which is not discussed herein further.

The controlling on the humidifier 4 or the mainframe 1 may be performed at the side of the ventilation-treatment-device mainframe, and may also be performed at the humidifier side.

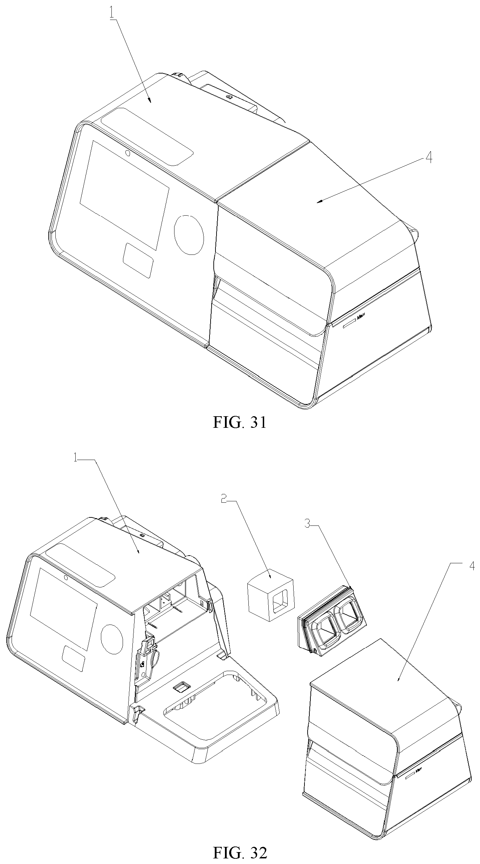

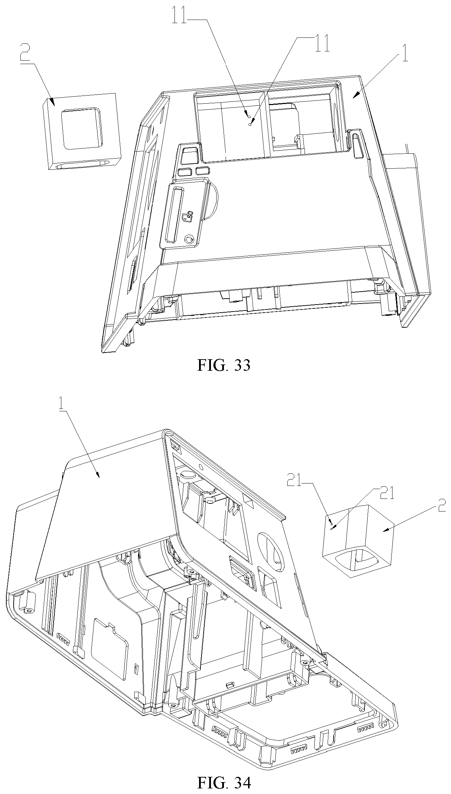

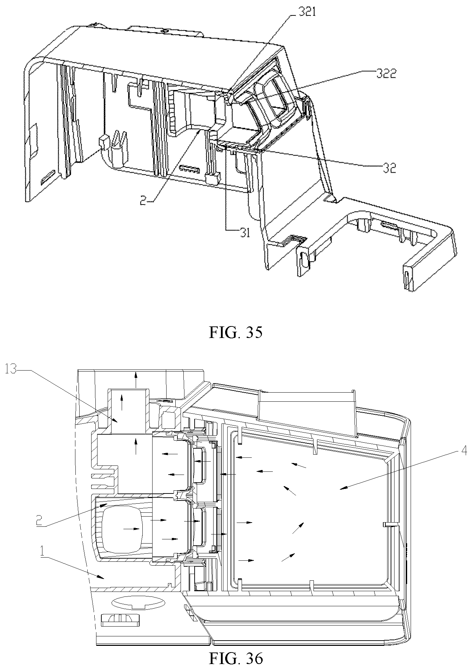

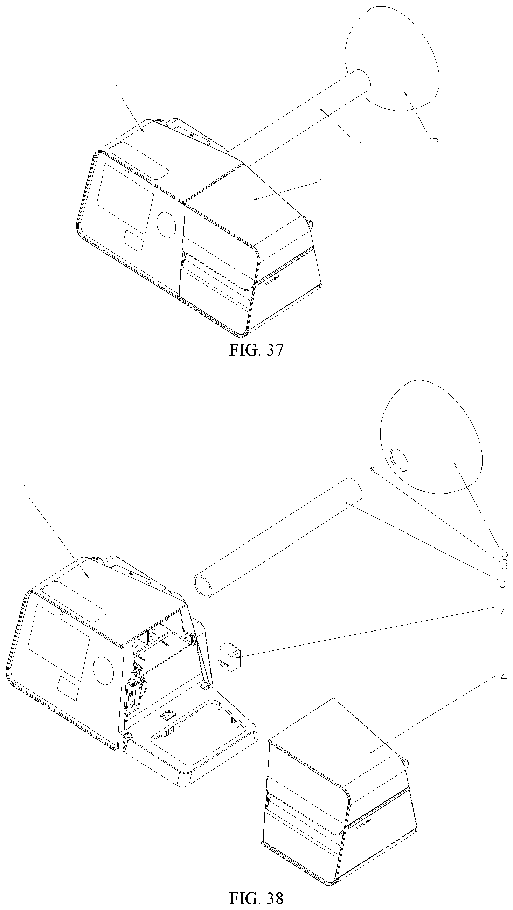

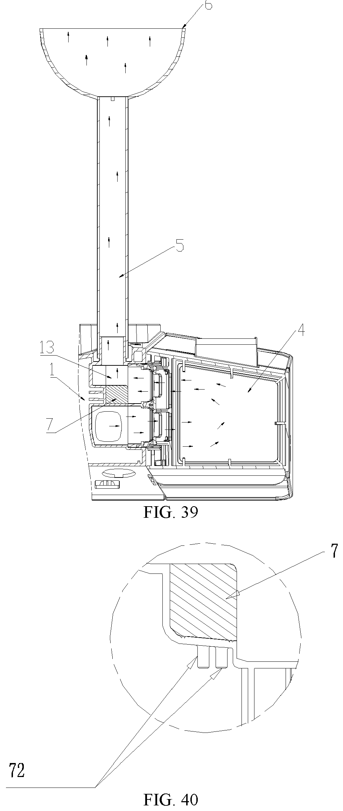

Referring to FIGs. 31-42, FIGs. 31-42 show another ventilation-treatment device according to the embodiments of the present disclosure. The ventilation-treatment device includes a mainframe 1 for generating a compressed gas and a humidifier 4 provided downstream of the mainframe 1 for humidifying the compressed gas. The ventilation-treatment device further includes a first heating body 2, wherein the first heating body 2 is located upstream of the humidifier 4.

In the ventilation-treatment device, the gas entering the humidifier 4 firstly passes through the first heating body 2 to be heated to have a higher temperature, and the high-temperature gas flow, when flowing through the humidifier 4, may bring away more water, thereby obtaining a more excellent humidification effect of a higher efficiency, which facilitates miniaturized ventilation-treatment devices to obtain an excellent humidification rate. Furthermore, when the first heating body 2 is provided, it may be not necessary to provide a heating unit at the bottom of the humidifier 4, thereby preventing the problem of, when the humidifier 4 is taken down from the mainframe for the liquid adding operation, exposure of the heating region, which might scald the user and the object covering it, to optimize the user experience, and have high safety and reliability.

In the embodiments shown in FIGs. 31 to 36, the mainframe 1 includes an outlet channel, the inlet of the humidifier 4 is in communication with the outlet channel of the mainframe 1, and the first heating body 2 is installed in the outlet channel of the mainframe 1. Accordingly, the gas generated by the mainframe 1 firstly passes through the first heating body 2 to be heated, and subsequently is discharged; in other words, the mainframe 1 generates the high-temperature gas. Optionally, the humidifier 4 may also be installed in the mainframe 1.

In some embodiments, a first-heating-body contact point 21 is formed on the first heating body 2, and the mainframe 1 is provided with a first contact-point connecting end 11. The first-heating-body contact point 21 is used to be connected to the first contact-point connecting end 11, so that the first-heating-body contact point 21 and the first contact-point connecting end 11 realize one or two of an electric connection and a signal connection therebetween.

For example, the controlling unit may be provided in the mainframe 1, and the mainframe 1 further includes an electric-power supplying terminal. By the connection between the first-heating-body contact point 21 and the first contact-point connecting end 11, the first heating body 2 may obtain electricity supply and the controlling instruction of the controlling unit from the mainframe 1.

Preferably, the ventilation-treatment device further includes a controlling unit and a first monitor. A signal outputting terminal of the first monitor is electrically connected to a signal inputting terminal of the controlling unit, and a signal outputting terminal of the controlling unit is electrically connected to the first heating body 2, whereby the controlling unit controls the heating of the first heating body 2 according to the monitored data of the first monitor. The first monitor is located downstream of the first heating body 2.

Accordingly, the monitor may be used to monitor the property of the gas downstream of the first heating body 2 in real time. For example, it monitors the temperature of the heated gas, and feeds back the monitoring result to the controlling unit, and the controlling unit receives the fed-back result, and subsequently, according to the fed-back result, sends a suitable controlling instruction to the first heating body 2, whereby the controlling unit controls the operation of the first heating body 2 according to the monitored data of the monitor, for example, the heat-emission efficacy of the first heating body 2. Accordingly, the operation of the first heating body 2 may be controlled more rationally and reasonably, to enable the gas delivered to the humidifier 4 to have a more reasonable temperature.

As an embodiment, the first monitor is located between the mainframe 1 and the humidifier 4, and the first monitor includes a temperature sensor. In other words, it is directly monitored whether the temperature of the gas entering the humidifier 4 is suitable, whereby a sufficient humidification rate inside the humidifier 2 may be obtained.

As another embodiment, the first monitor may be located downstream of the humidifier 4, and the first monitor includes one or a combination of a temperature sensor, a humidity sensor, a gas-flow-rate sensor, a pressure sensor and an oxyhemoglobin-saturation sensor. For example, by monitoring the humidity and/or the temperature of the gas flow discharged from the humidifier 4, the operation state of the first heating body 2 is adjusted, to increase or reduce the heat-emission efficacy of the first heating body 2, whereby the gas flow discharged from the humidifier 4 has a suitable humidity and/or temperature. The first monitor may be close to the patient side, whereby, by monitoring and feeding back the signals and controlling the first heating body 2 to operate, both of the temperature and/or the humidity of the gas flow at the patient side may be more suitable, to optimize the user experience. For example, the first monitor may be located in a gas returning chamber 13 downstream of the humidifier 4.

Particularly, in the present embodiment, the first heating body 2 is provided with an elastic convex rib at the periphery, so that the first heating body 2 is interference-fitted inside the outlet channel. A first-heating-body channel is formed on the first heating body 2, and the first-heating-body channel is in communication with the outlet of the mainframe 1 and the inlet of the humidifier 4. Accordingly, the first heating body 2 is replaceable, to facilitate repairing, maintenance and replacement.

Further particularly, the installation channel has a rectangular cross-section, the first heating body 2 is correspondingly generally of a rectangular shape, the elastic convex rib circumferentially surrounds the first heating body 2 to form a ring, and the first-heating-body contact point 21 on the first heating body 2 is located on the end surface of the side of the first heating body 2 that faces the mainframe 1.

In the present embodiment, a first-heating-body channel is formed on the first heating body 2, and the first-heating-body channel is in communication with the outlet of the mainframe 1 and the inlet of the humidifier 4, whereby all of the gases passing through the installation channel pass through the first-heating-body channel, to increase the heating efficiency. Furthermore, it can be understood that the quantity of the first-heating-body channel may be one or more. The first heating body 2 may include a heating wire, an electromagnetic-induction unit and so on, to be able to generate heat.

Furthermore, the ventilation-treatment device further includes a switching unit 3 provided between the mainframe 1 and the humidifier 4, the switching unit 3 has a switching channel in communication with the mainframe 1 and the humidifier 4, and the first heating body 2 is provided inside the switching channel. Optionally, as an embodiment, the switching unit 3 includes a hard frame 31 and a soft body 32, the hard frame 31 includes a first side facing the mainframe 1 and a second side facing the humidifier 4, and the soft body 32 is provided on the second side.

Preferably, the first side of the hard frame 31 is used to abut the first heating body 2. In other words, after installed in place, the first side of a hardness of the hard frame 31 abuts the first heating body 2, thereby restricting the first heating body 2 inside the installation channel, to prevent displacement of the first heating body 2, which affects the normal usage.

Optionally, the switching unit 3 includes a switching channel extending from the first side to the second side, to communicate the outlet of the mainframe 1 and the inlet of the humidifier 4, whereby the gas flow may flow from the mainframe 1 into the humidifier 4 via the switching channel. A first convex rib 321 and a second convex rib 322 are formed on the soft body 32. The first convex rib 321 protrudes out of the edge of the second side toward the mainframe 1. The second convex rib 322 surrounds an opening of the switching channel on the second side. The soft body 32 may be silica gel, rubber and so on. The first convex rib 321 is used to enable the switching unit 3 to be installed to the mainframe 1 by interference fitting. The second convex rib 322 is used to be engaged with the humidifier 4 to form a sealing.

Furthermore, in the illustrated embodiments, another switching channel is formed in the switching unit 3, to communicate a reflux opening of the mainframe 1 and the outlet of the humidifier 4, wherein the reflux opening is the inlet of the gas returning chamber 13.

In some embodiments, the ventilation-treatment device may further include a connecting pipeline 5 and a second heating body 7, one end of the connecting pipeline 5 is in communication with the outlet of the humidifier 4, and the second heating body 7 is located at the outlet of the humidifier 4 or located between the humidifier 4 and the connecting pipeline 5.

Accordingly, by providing the first heating body 2 and the second heating body 7 upstream of the connecting pipeline 5, the gas obtained after the humidification in the connecting pipeline 5 may have a higher temperature, and be transported in the connecting pipeline 5 while maintaining the higher temperature, thereby alleviating the problem that the temperature of the gas obtained after the humidification decreases in the connecting pipeline 5 or even condensed water is generated.

Preferably, the ventilation-treatment device further includes a patient side 6, a controlling unit and a second monitor 8. The other end of the connecting pipeline 5 is connected to the inlet of the patient side 6. The second monitor 8 is provided at the side of the connecting pipeline 5 close to the patient side 6 or is provided at the patient side 6. A signal outputting terminal of the second monitor 8 is electrically connected to a signal inputting terminal of the controlling unit, and a signal outputting terminal of the controlling unit is electrically connected to the second heating body 7, whereby the controlling unit controls the heating of the second heating body 7 according to the monitored data of the second monitor 8.

Accordingly, the second monitor 8 may be used to monitor in real time the property of the gas obtained after the humidification that is to be delivered to the patient side 6. For example, it monitors the temperature, the humidity and so on of the gas obtained after the humidification, and feeds back the monitoring result to the controlling unit, and the controlling unit receives the fed-back result, and subsequently, according to the fed-back result, sends a suitable controlling instruction to the second heating body 7 or to both of the first heating body 2 and the second heating body 7, whereby the controlling unit controls the operation of the relevant heating bodies according to the monitored data of the second monitor 8, for example, the heat-emission efficacy of the second heating body 7. Accordingly, the operation of the relevant heating bodies may be controlled more rationally and reasonably, to enable the gas obtained after the humidification that is delivered to the patient side 6 to be more suitable for the usage by the patient.

The second monitor 8 may include one or a combination of a temperature sensor, a humidity sensor, a gas-flow-rate sensor, a pressure sensor and an oxyhemoglobin-saturation sensor. The position of the second monitor 8 is, preferably, close to the patient side 6, for example, located at the tip of the other end of the connecting pipeline 5 or a front end of the patient side 6, whereby more accurate data may be collected, to feed back to the controlling unit in time, obtain a higher user usability, and prevent damaging of the device and uncomfortableness of the patient. Moreover, the patient side 6 may be a face mask.

Moreover, the installation position of the second heating body 7 may have multiple options according to practical situations. For example, the ventilation-treatment device includes a mainframe 1, the humidifier 4 is installed to the mainframe 1, a gas returning chamber 13 is provided in the mainframe 1, the two ends of the gas returning chamber 13 are connected to the outlet of the humidifier 4 and one end of the connecting pipeline 5, and the second heating body 7 is installed in the gas returning chamber 13. Accordingly, the gas obtained after the humidification that is discharged from the outlet of the humidifier 4, when passing through the gas returning chamber 13, is heated again by the second heating body 7, subsequently discharged again at a higher temperature into the connecting pipeline 5, and finally delivered to the patient, which effectively prevents generation of condensed water in the connecting pipeline 5.

Regarding the installation of the second heating body 7 in the mainframe 1, preferably, a clipping rib 14 protrudes out of one of the second heating body 7 and the gas returning chamber 13, and a slot 71 matching with the clipping rib 14 is formed on the other, whereby the second heating body 7 may be installed in the gas returning chamber 13 by snap fitting. The slot 71 may be formed in the form of a socket, the fixing of the second heating body 7 in the mainframe 1 may be realized by inserting the clipping rib 14 into the socket, and the second heating body 7 may be taken down when it requires maintenance, repairing or replacement. In addition, alternatively, the connection between the second heating body 7 and the mainframe 1 or the humidifier 4 may also be by means of embedding or direct integral formation by using a mold.

In the present embodiment, the mainframe 1 is provided with a second contact-point connecting end 12, a second-heating-body contact point 72 is formed on the second heating body 7, and the second-heating-body contact point 72 is connected to the second contact-point connecting end 12, whereby the second-heating-body contact point 72 and the second contact-point connecting end 12 realize one or two of an electric connection and a signal connection therebetween.

For example, the controlling unit may be provided in the mainframe 1, and the mainframe 1 further includes an electric-power supplying terminal. By the connection between the second-heating-body contact point 72 and the second contact-point connecting end 12, the second heating body 7 may obtain electricity supply and the controlling instruction of the controlling unit from the mainframe 1.

Optionally, in order to prevent water vapor from affecting the second-heating-body contact point 72 and the second contact-point connecting end 12, the second heating body 7 is further provided with a sealing rib 73 surrounding the second-heating-body contact point 72. The sealing rib 73 may be of any suitable shape. When the second heating body 7 is installed to the mainframe 1, the sealing rib 73 is hermetically engaged with the surface of the mainframe 1 (the inner surface of the gas returning chamber 13), to provide a closed environment of the second-heating-body contact point 72 and the second contact-point connecting end 12.

In addition, optionally, the second heating body 7 includes a second-heating-body main body and a covering-adhesive layer covering the outer surface of the second-heating-body main body. The second-heating-body main body may include a heating wire, an electromagnetic-induction heat emitting unit and so on. The covering-adhesive layer may close and protect the second-heating-body main body, to prevent it from being affected by water vapor. The second heating body 7 may be configured so that, in the normal usage, its heat emission does not cause risks such as touching scald.

In some other embodiments, the humidifier 4 may be a heat and moisture exchanger. The humidifier 4 may be installed to the mainframe 1. The mainframe 1 is provided with a third heating body, and the humidifier 4 is installed over the third heating body. The third heating body heats the bottom of the humidifier 4, and the humidifying liquid inside the humidifier 4 is heated, whereby water vapor is more easily formed and brought away by the gas, which facilitates to increase the humidification rate.

Regarding the above embodiments of the ventilation-treatment device, because the part of its humidifier is substantially similar to the embodiments of the humidifier, the relative parts may refer to the description on the embodiments of the humidifier.

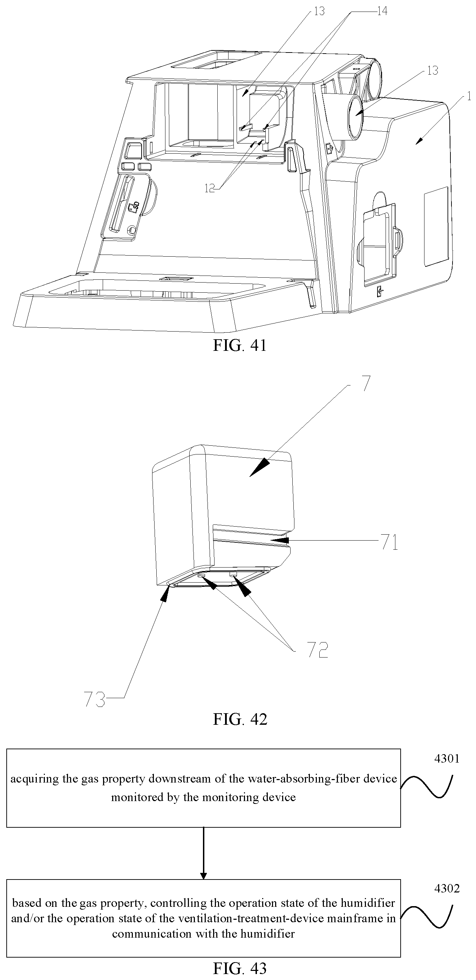

Referring to FIG. 43, an embodiment of the present disclosure further provides a controlling method of the humidifier according to the above embodiments, wherein the method includes:
Step 4301: acquiring the gas property downstream of the water-absorbing-fiber device monitored by the monitoring device; and
Step 4302: based on the gas property, controlling the operation state of the humidifier and/or the operation state of the ventilation-treatment-device mainframe in communication with the humidifier.

The controlling method of the humidifier according to the embodiments of the present disclosure, by acquiring the gas property downstream of the water-absorbing-fiber device inside the humidifier monitored by the monitoring device, and, based on the monitored gas property, controlling the operation state of the humidifier and/or the ventilation-treatment-device mainframe, may treat liquid insufficiency inside the humidifier in time, to achieve the purpose of preventing dry heating. Accordingly, dry heating of the humidifier may be effectively prevented without the patient or the user always paying attention to the residual amount of the humidifying liquid, which prevents component damage and patient ambustion caused by dry heating of the humidifier, thereby prolonging the service life of the product, and improving the safety and the reliability.

Optionally, the step 4302 includes:
when the gas property is in the first abnormal state, reducing the heating power of the humidifier; and/or
when the gas property is in the second abnormal state, shutting-down the heating function of the humidifier or stopping the operating state of the ventilation-treatment-device mainframe.

In this case, when the monitored gas property is in the first abnormal state, the first abnormal state indicates that the residual amount of the humidifying liquid inside the humidifying chamber of the humidifier is lower than a first water level, the first water level represents that the remaining humidifying liquid may still support the humidifier to continuously operate, but has already been consumed partially, then the heating power of the humidifier is reduced, to prolong the usage duration of the ventilation-treatment device, to prevent the humidifying liquid from being quickly consumed off. When the monitored gas property is in the second abnormal state, the second abnormal state indicates that the residual amount of the humidifying liquid inside the humidifying chamber of the humidifier is lower than a second water level, the second water level represents that the remaining humidifying liquid has already been insufficient to support the humidifier to continuously operate, or even there has already been no humidifying liquid, then the heating function of the humidifier is shut down or the operating state of the ventilation-treatment-device mainframe is stopped, to prevent dry heating.

What type of the abnormal states the gas property is in may refer to the above description on the embodiments of the humidifier side, which is not discussed herein further.

The controlling method of the humidifier according to the embodiments of the present disclosure may be implemented at the humidifier side, and may also be implemented at the side of the ventilation-treatment-device mainframe, which is not limited herein.

It should be noted that, in the present text, relation terms such as first and second are merely intended to distinguish one entity or operation from another entity or operation, and that does not necessarily require or imply that those entities or operations have therebetween any such actual relation or order. Furthermore, the terms "include", "comprise" or any variants thereof are intended to cover non-exclusive inclusions, so that processes, articles or devices that include a series of elements do not only include those elements, but also include other elements that are not explicitly listed, or include the elements that are inherent to such processes, articles or devices. Unless further limitation is set forth, an element defined by the wording "comprising a ..." does not exclude additional same element in the process, article or device comprising the element.

The embodiments of the description are described in the mode of correlation, the same or similar parts of the embodiments may refer to each other, and each of the embodiments emphatically describes the differences from the other embodiments. Especially, regarding the system embodiments, because they are substantially similar to the process embodiments, they are described simply, and the related parts may refer to the description on the process embodiments.

## Claims

1. A humidifier (4), comprising a housing (41), the housing (41) being provided with a gas inlet (43), a gas outlet (44) and a humidifying chamber (45) for containing a humidifying liquid, an external gas entering the housing (41) via the gas inlet (43), flowing through the humidifying chamber (45), and being discharged via the gas outlet (44), wherein the humidifier (4) further comprises:
a water-absorbing-fiber device, wherein the water-absorbing-fiber device is provided inside the humidifying chamber (45), and extends in a longitudinal direction of the humidifying chamber (45), the water-absorbing-fiber device comprises a first cross section (421), and the first cross section (421) intersects with a gas-flow direction of the external gas after the external gas enters the humidifying chamber (45); and
a monitoring device (9), wherein the monitoring device (9) is provided downstream of the water-absorbing-fiber device in the gas-flow direction, and is for monitoring a gas property downstream of the water-absorbing-fiber device,
**characterized in that**
the water-absorbing-fiber device comprises:
at least two water adsorbing filter elements (42), wherein the at least two water adsorbing filter elements (42) are arranged inside the humidifying chamber (45) sequentially in the gas-flow direction, and each of the water adsorbing filter elements (42) has the first cross section (421), and
the monitoring device (9) comprises: at least two monitors (91), wherein the monitors (91) are provided downstream of different water adsorbing filter elements (42) in the gas-flow direction.

2. The humidifier (4) according to claim 1, wherein, at least part of the water adsorbing filter elements (42) incline relatively to a transverse direction of the humidifying chamber (45), and extend in an inclined direction from a top to a bottom of the humidifying chamber (45) to form the first cross section (421).

3. The humidifier (4) according to claim 2, wherein, the water adsorbing filter elements (42) inclining relatively to the transverse direction of the humidifying chamber (45) comprise a plurality of first water adsorbing filter elements (422) and a plurality of second water adsorbing filter elements (423) that are arranged alternately inside the humidifying chamber (45) in the gas-flow direction, and directions of inclining relative to the transverse direction of the humidifying chamber (45) of the first water adsorbing filter elements (422) and the second water adsorbing filter elements (423) are opposite.

4. The humidifier (4) according to claim 3, wherein, neighboring instances of the first water adsorbing filter elements (422) and the second water adsorbing filter elements (423) form a V shape therebetween.

5. The humidifier (4) according to claim 3, wherein, a first water adsorbing filter element (424) disposed at a first position in the gas-flow direction and a second water adsorbing filter element (425) disposed at a last position in the gas-flow direction individually extend in the direction from the top to the bottom of the humidifying chamber (45) to form the first cross section (421); and
the first water adsorbing filter elements (422) and the second water adsorbing filter elements (423) are arranged alternately between the first water adsorbing filter element (424) disposed at the first position and the second water adsorbing filter element (425) disposed at the last position.

6. The humidifier (4) according to any one of claims 2 to 5, wherein, lengths of extension in the direction from the top to the bottom of the humidifying chamber (45) of part or all of the water adsorbing filter elements (42) are unequal.

7. The humidifier (4) according to claim 6, wherein, the lengths of the extension in the direction from the top to the bottom of the humidifying chamber (45) of the part or all of the water adsorbing filter elements (42) decrease or increase sequentially in an arrangement direction.

8. The humidifier (4) according to any one of claims 3 to 7, wherein, each two neighboring instances of the first water adsorbing filter elements (422) and the second water adsorbing filter elements (423) are grouped as one group, and each of the remaining individual water adsorbing filter elements (42) is grouped as one group; and
lengths of extension in the direction from the top to the bottom of the humidifying chamber (45) of different groups of the water adsorbing filter elements (42) decrease or increase sequentially in the arrangement direction.

9. The humidifier (4) according to any one of claims 2 to 7, wherein, the bottom of the humidifying chamber (45) inclines, and each of the water adsorbing filter elements (42) extends in an extension direction from the top to the bottom of the humidifying chamber (45).

10. The humidifier (4) according to any one of claims 1 to 9, wherein, each of the water adsorbing filter elements (42) comprises a water-absorbing-fiber matrix (426) and a border frame (427) for fixing the water-absorbing-fiber matrix (426); and
an installation region (453) for installing the water adsorbing filter elements (42) is provided inside the humidifying chamber (45); and/or
the humidifier (4) further comprises:
a first controller, wherein the first controller is electrically connected to the monitoring device (9), and is for, based on the gas property monitored by the monitoring device (9), controlling an operation state of the humidifier (4), and/or, an operation state of a ventilation-treatment-device mainframe (1) in communication with the humidifier (4).

11. The humidifier (4) according to any one of claims 1 to 10, wherein, the humidifier (4) further comprises:
a water tank (10), wherein the water tank (10) is provided at a bottom of the housing (41), a first communicating component is provided at a top of the water tank (10), a second communicating component in cooperation with the first communicating component is provided at the bottom of the housing (41), and the housing (41) and the water tank (10) are communicated by the cooperation between the first communicating component and second communicating component.

12. A ventilation-treatment device (100), comprising a mainframe (1) for generating a gas of a preset pressure, the mainframe (1) comprising a gas outlet channel, wherein the ventilation-treatment device (100) further comprises: the humidifier (4) according to any one of claims 1-11, and the gas outlet channel of the mainframe (1) is in communication with the gas inlet (43) of the humidifier (4).

13. The ventilation-treatment device (100) according to claim 12 further comprises:
a second controller, wherein the second controller is electrically connected to the monitoring device (9), is electrically connected to the mainframe (1) and/or the humidifier (4), and is for, based on the gas property monitored by the monitoring device (9), controlling an operation state of the mainframe (1) and/or the humidifier (4); and/or
the ventilation-treatment device (100) further comprises:
a first heating body (2), wherein the first heating body (2) is located upstream of the humidifier (4).

## Patentansprüche

1. Ein Befeuchter (4), aufweisend ein Gehäuse (41), wobei das Gehäuse (41) mit einem Gaseinlass (43), einem Gasauslass (44) und einer Befeuchtungskammer (45) zur Aufnahme einer Befeuchtungsflüssigkeit versehen ist, wobei ein externes Gas in das Gehäuse (41) über den Gaseinlass (43) eintritt, durch die Befeuchtungskammer (45) strömt und über den Gasauslass (44) abgeführt wird, wobei der Befeuchter (4) ferner aufweist:
eine wasserabsorbierende Faservorrichtung, wobei die wasserabsorbierende Faservorrichtung innerhalb der Befeuchtungskammer (45) vorgesehen ist und sich in einer Längsrichtung der Befeuchtungskammer (45) erstreckt, wobei die wasserabsorbierende Faservorrichtung einen ersten Querschnitt (421) aufweist und der erste Querschnitt (421) sich mit einer Gasströmungsrichtung des externen Gases schneidet, nachdem das externe Gas in die Befeuchtungskammer (45) eintritt; und
eine Überwachungsvorrichtung (9), wobei die Überwachungsvorrichtung (9) stromabwärts der wasserabsorbierenden Faservorrichtung in der Gasströmungsrichtung vorgesehen ist, und zur Überwachung einer Gaseigenschaft stromabwärts der wasserabsorbierenden Faservorrichtung dient,
**dadurch gekennzeichnet, dass**
die wasserabsorbierende Faservorrichtung aufweist:
mindestens zwei wasserabsorbierende Filterelemente (42), wobei die mindestens zwei wasserabsorbierenden Filterelemente (42) innerhalb der Befeuchtungskammer (45) in Gasströmungsrichtung aufeinanderfolgend angeordnet sind, und jedes der wasserabsorbierenden Filterelemente (42) den ersten Querschnitt (421) aufweist, und
die Überwachungsvorrichtung (9) aufweist: mindestens zwei Monitore (91), wobei die Monitore (91) stromabwärts von verschiedenen wasseradsorbierenden Filterelementen (42) in der Gasströmungsrichtung vorgesehen sind.

2. Der Befeuchter (4) nach Anspruch 1, wobei zumindest ein Teil der wasseradsorbierenden Filterelemente (42) relativ zu einer Querrichtung der Befeuchtungskammer (45) geneigt ist und sich in einer geneigten Richtung von einer Oberseite zu einer Unterseite der Befeuchtungskammer (45) erstreckt, um den ersten Querschnitt (421) zu bilden.

3. Der Befeuchter (4) nach Anspruch 2, wobei die relativ zur Querrichtung der Befeuchtungskammer (45) geneigten wasseradsorbierenden Filterelemente (42) eine Vielzahl von ersten wasseradsorbierenden Filterelementen (422) und eine Vielzahl von zweiten wasseradsorbierenden Filterelementen (423) aufweisen, die abwechselnd innerhalb der Befeuchtungskammer (45) in der Gasströmungsrichtung angeordnet sind, und die Neigungsrichtungen der ersten wasseradsorbierenden Filterelemente (422) und der zweiten wasseradsorbierenden Filterelemente (423) in Bezug auf die Querrichtung der Befeuchtungskammer (45) entgegengesetzt sind.

4. Der Befeuchter (4) nach Anspruch 3, wobei benachbarte Stellen der ersten wasseradsorbierenden Filterelemente (422) und der zweiten wasseradsorbierenden Filterelemente (423) zwischen sich eine V-Form bilden.

5. Der Befeuchter (4) nach Anspruch 3, wobei ein erstes wasseradsorbierendes Filterelement (424), das an einer ersten Position in der Gasströmungsrichtung angeordnet ist, und ein zweites wasseradsorbierendes Filterelement (425), das an einer letzten Position in der Gasströmungsrichtung angeordnet ist, sich einzeln in der Richtung von der Oberseite zum Boden der Befeuchtungskammer (45) erstrecken, um den ersten Querschnitt (421) zu bilden; und
die ersten wasseradsorbierenden Filterelemente (422) und die zweiten wasseradsorbierenden Filterelemente (423) abwechselnd zwischen dem ersten wasseradsorbierenden Filterelement (424), das an der ersten Position angeordnet ist, und dem zweiten wasseradsorbierenden Filterelement (425), das an der letzten Position angeordnet ist, angeordnet sind.

6. Der Befeuchter (4) nach einem der Ansprüche 2 bis 5, wobei die Ausdehnungslängen in Richtung von der Oberseite zur Unterseite der Befeuchtungskammer (45) eines Teils oder aller wasseradsorbierenden Filterelemente (42) ungleich sind.

7. Der Befeuchter (4) nach Anspruch 6, wobei die Längen der Ausdehnung in der Richtung von der Oberseite zur Unterseite der Befeuchtungskammer (45) des Teils oder aller wasseradsorbierenden Filterelemente (42) in einer Anordnungsrichtung aufeinanderfolgend abnehmen oder zunehmen.

8. Der Befeuchter (4) nach einem der Ansprüche 3 bis 7, wobei jeweils zwei benachbarte Exemplare der ersten wasseradsorbierenden Filterelemente (422) und der zweiten wasseradsorbierenden Filterelemente (423) als eine Gruppe gruppiert sind und jedes der übrigen einzelnen wasseradsorbierenden Filterelemente (42) als eine Gruppe gruppiert ist; und
die Erstreckungslängen in der Richtung von der Oberseite zur Unterseite der Befeuchtungskammer (45) von verschiedenen Gruppen der wasseradsorbierenden Filterelemente (42) in der Anordnungsrichtung nacheinander abnehmen oder zunehmen.

9. Der Befeuchter (4) nach einem der Ansprüche 2 bis 7, wobei der Boden der Befeuchtungskammer (45) geneigt ist und jedes der wasseradsorbierenden Filterelemente (42) sich in einer Ausdehnungsrichtung von der Oberseite zum Boden der Befeuchtungskammer (45) erstreckt.

10. Der Befeuchter (4) nach einem der Ansprüche 1 bis 9, wobei jedes der wasserabsorbierenden Filterelemente (42) eine wasserabsorbierende Fasermatrix (426) und einen Rahmen (427) zur Befestigung der wasserabsorbierenden Fasermatrix (426) aufweist; und
innerhalb der Befeuchtungskammer (45) ein Installationsbereich (453) zum Installieren der wasserabsorbierenden Filterelemente (42) vorgesehen ist; und/oder
der Befeuchter (4) ferner aufweist:
eine erste Steuereinheit, wobei die erste Steuereinheit elektrisch mit der Überwachungsvorrichtung (9) verbunden ist und dazu dient, auf der Grundlage der von der Überwachungsvorrichtung (9) überwachten Gaseigenschaft einen Betriebszustand des Befeuchters (4) und/oder einen Betriebszustand eines mit dem Befeuchter (4) in Verbindung stehenden Hauptcomputers (1) eines Belüftungs-Behandlungsgeräts zu steuern.

11. Der Befeuchter (4) nach einem der Ansprüche 1 bis 10, wobei der Befeuchter (4) ferner aufweist:
einen Wassertank (10), wobei der Wassertank (10) an einem Boden des Gehäuses (41) vorgesehen ist, eine erste kommunizierende Komponente an einer Oberseite des Wassertanks (10) vorgesehen ist, eine zweite kommunizierende Komponente, die mit der ersten kommunizierenden Komponente zusammenwirkt, am Boden des Gehäuses (41) vorgesehen ist, und das Gehäuse (41) und der Wassertank (10) durch das Zusammenwirken zwischen der ersten kommunizierenden Komponente und der zweiten kommunizierenden Komponente miteinander verbunden sind.

12. Eine Beatmungsvorrichtung (100), die einen Hauptrahmen (1) zum Erzeugen eines Gases mit einem voreingestellten Druck aufweist, wobei der Hauptrahmen (1) einen Gasauslasskanal aufweist, wobei die Beatmungsvorrichtung (100) ferner aufweist: den Befeuchter (4) nach einem der Ansprüche 1 bis 11, und der Gasauslasskanal des Hauptrahmens (1) mit dem Gaseinlass (43) des Befeuchters (4) in Verbindung steht.

13. Die Beatmungsvorrichtung (100) nach Anspruch 12 weist ferner auf:
eine zweite Steuereinheit, wobei die zweite Steuereinheit elektrisch mit der Überwachungsvorrichtung (9) verbunden ist, elektrisch mit dem Hauptrahmen (1) und/oder dem Befeuchter (4) verbunden ist und dazu dient, basierend auf der von der Überwachungsvorrichtung (9) überwachten Gaseigenschaft einen Betriebszustand des Hauptrahmens (1) und/oder des Befeuchters (4) zu steuern; und/oder
die Beatmungsvorrichtung (100) weiterhin aufweist:
einen ersten Heizkörper (2), wobei der erste Heizkörper (2) stromaufwärts des Befeuchters (4) angeordnet ist.

## Revendications

1. Humidificateur (4), comprenant un logement (41), le logement (41) étant pourvu d'un orifice d'entrée de gaz (43), d'un orifice de sortie de gaz (44) et d'une chambre d'humidification (45) pour contenir un liquide humidifiant, un gaz externe entrant dans le logement (41) par l'intermédiaire de l'orifice d'entrée de gaz (43), s'écoulant à travers la chambre d'humidification (45), et étant évacué par l'intermédiaire de l'orifice de sortie de gaz (44), dans lequel l'humidificateur (4) comprend en outre :
un dispositif à fibres absorbant l'eau, dans lequel le dispositif à fibres absorbant l'eau est disposé à l'intérieur de la chambre d'humidification (45), et s'étend dans un sens longitudinal de la chambre d'humidification (45), le dispositif à fibres absorbant l'eau comprend une première section transversale (421), et la première section transversale (421) entre en intersection avec un sens d'écoulement de gaz du gaz externe après que le gaz externe entre dans la chambre d'humidification (45) ; et
un dispositif de surveillance (9), dans lequel le dispositif de surveillance (9) est disposé en aval du dispositif à fibres absorbant l'eau dans le sens d'écoulement de gaz, et sert à surveiller une propriété du gaz en aval du dispositif à fibres absorbant l'eau,
**caractérisé en ce que**
le dispositif à fibres absorbant l'eau comprend :
moins deux éléments filtres adsorbant l'eau (42), dans lequel les au moins deux éléments filtres adsorbant l'eau (42) sont agencés à l'intérieur de la chambre d'humidification (45) séquentiellement dans le sens d'écoulement de gaz, et chacun des éléments filtres adsorbant l'eau (42) présente la première section transversale (421), et
le dispositif de surveillance (9) comprend : au moins deux moniteurs (91), dans lequel les moniteurs (91) sont disposés en aval des éléments filtres adsorbant l'eau (42) différents dans le sens d'écoulement de gaz.

2. Humidificateur (4) selon la revendication 1, dans lequel, au moins une partie des éléments filtres adsorbant l'eau (42) s'incline relativement à un sens transversal de la chambre d'humidification (45), et s'étend dans un sens incliné depuis un sommet vers un fond de la chambre d'humidification (45) pour former la première section transversale (421).

3. Humidificateur (4) selon la revendication 2, dans lequel, les éléments filtres adsorbant l'eau (42) s'inclinant relativement au sens transversal de la chambre d'humidification (45) comprennent une pluralité de premiers éléments filtres adsorbant l'eau (422) et une pluralité de seconds éléments filtres adsorbant l'eau (423) qui sont agencés alternativement à l'intérieur de la chambre d'humidification (45) dans le sens d'écoulement de gaz, et des sens d'inclinaison par rapport au sens transversal de la chambre d'humidification (45) des premiers éléments filtres adsorbant l'eau (422) et les seconds éléments filtres adsorbant l'eau (423) sont opposés.

4. Humidificateur (4) selon la revendication 3, dans lequel, des cas de mise en place adjacente des premiers éléments filtres adsorbant l'eau (422) et des seconds éléments filtres adsorbant l'eau (423) réalisent une forme de « V » entre eux.

5. Humidificateur (4) selon la revendication 3, dans lequel, un premier élément filtre adsorbant l'eau (424) disposé à une première position dans le sens d'écoulement de gaz et un second élément filtre adsorbant l'eau (425) disposé à une dernière position dans le sens d'écoulement de gaz s'étendent individuellement dans le sens depuis le sommet vers le fond de la chambre d'humidification (45) pour former la première section transversale (421) ; et
les premiers éléments filtres adsorbant l'eau (422) et les seconds éléments filtres adsorbant l'eau (423) sont agencés alternativement entre le premier élément filtre adsorbant l'eau (424) disposé à la première position et le second élément filtre adsorbant l'eau (425) disposé à la dernière position.

6. Humidificateur (4) selon l'une quelconque des revendications 2 à 5, dans lequel, des longueurs d'extension dans le sens depuis le sommet vers le fond de la chambre d'humidification (45) d'une partie ou de tous les éléments filtres adsorbant l'eau (42) sont inégales.

7. Humidificateur (4) selon la revendication 6, dans lequel, les longueurs de l'extension dans le sens depuis le sommet vers le fond de la chambre d'humidification (45) de la partie ou de tous les éléments filtres adsorbant l'eau (42) baissent ou augmentent séquentiellement dans un sens d'agencement.

8. Humidificateur (4) selon l'une quelconque des revendications 3 à 7, dans lequel, chacun des deux cas de mise en place adjacente des premiers éléments filtres adsorbant l'eau (422) et des seconds éléments filtres adsorbant l'eau (423) sont groupés en un groupe, et chacun des éléments filtres adsorbant l'eau (42) individuels restants est regroupé comme un seul groupe ; et
des longueurs d'extension dans le sens depuis le sommet vers le fond de la chambre d'humidification (45) de groupes différents des éléments filtres adsorbant l'eau (42) baissent ou augmentent séquentiellement dans le sens d'agencement.

9. Humidificateur (4) selon l'une quelconque des revendications 2 à 7, dans lequel, le fond de la chambre d'humidification (45) s'incline, et chacun des éléments filtres adsorbant l'eau (42) s'étend dans un sens d'extension depuis le sommet vers le fond de la chambre d'humidification (45).

10. Humidificateur (4) selon l'une quelconque des revendications 1 à 9, dans lequel, chacun des éléments filtres adsorbant l'eau (42) comprend une matrice de fibres absorbant l'eau (426) et un cadre formant bordure (427) pour fixer la matrice de fibres absorbant l'eau (426) ; et
une région d'installation (453) pour installer les éléments filtres adsorbant l'eau (42) est prévue à l'intérieur de la chambre d'humidification (45) ; et/ou
l'humidificateur (4) comprend en outre :
un premier dispositif de commande, dans lequel le premier dispositif de commande est électriquement connecté au dispositif de surveillance (9), et est destiné, sur la base de la propriété du gaz surveillée par le dispositif de surveillance (9), à commander un état de fonctionnement de l'humidificateur (4), et/ou, un état de fonctionnement d'un cadre principal (1) du dispositif de ventilation-traitement en communication avec l'humidificateur (4).

11. Humidificateur (4) selon l'une quelconque des revendications 1 à 10, dans lequel, l'humidificateur (4) comprend en outre :
une cuve d'eau (10), dans lequel la cuve d'eau (10) est disposée au niveau d'un fond du logement (41), un premier composant de communication est disposé au niveau d'un sommet de la cuve d'eau (10), un second composant de communication en coopération avec le premier composant de communication est disposé au fond du logement (41), et le logement (41) et la cuve d'eau (10) communiquent par la coopération entre le premier composant de communication et le second composant de communication.

12. Dispositif de ventilation-traitement (100), comprenant un cadre principal (1) pour générer un gaz d'une pression prédéfinie, le cadre principal (1) comprenant un canal d'orifice de sortie de gaz, dans lequel le dispositif de ventilation-traitement (100) comprend en outre : l'humidificateur (4) selon l'une quelconque des revendications 1 à 11, et le canal **d'orifice** de sortie de gaz du cadre principal (1) se trouve en communication avec l'orifice d'entrée de gaz (43) de l'humidificateur (4).

13. Dispositif de ventilation-traitement (100) selon la revendication 12 qui comprend en outre :
un second dispositif de commande, dans lequel le second dispositif de commande est électriquement connecté au dispositif de surveillance (9), est électriquement connecté au cadre principal (1) et/ou à l'humidificateur (4), et est destiné, sur la base de la propriété du gaz surveillée par le dispositif de surveillance (9), à commander un état de fonctionnement du cadre principal (1) et/ou de l'humidificateur (4) ; et/ou
le dispositif de ventilation-traitement (100) comprend en outre :
un premier corps chauffant (2), dans lequel le premier corps chauffant (2) est localisé en amont de l'humidificateur (4).
